# EUROPEAN PATENT APPLICATION

(11) **EP 3 141 263 A1**
(43) Date of publication of application: **15.03.2017**
(21) Application number: 15788881.9
(22) Date of filing: 09.03.2015
(51) Int. Cl.: A61K 47/32, A61K 47/34

(54) **METAL-ORGANIC FRAMEWORK/STIMULUS-RESPONSIVE POLYMER COMPOSITE CAPABLE OF CONTROLLING RELEASE OF GUEST**

(30) Priority: 08.05.2014 JP 2014097006
(71) Applicant: National University Corporation Hokkaido University, Sapporo-shi, Hokkaido 060-0808 (JP); Nissan Chemical Industries, Ltd., Chiyoda-ku Tokyo 101-0054 (JP)
(72) Inventor: SADA, Kazuki, Sapporo-shi Hokkaido 060-0808 (JP); KOKADO, Kenta, Sapporo-shi Hokkaido 060-0808 (JP); NAGATA, Shunjiro, Sapporo-shi Hokkaido 060-0808 (JP)
(74) Representative: f & e patent
(86) International application number: PCT/JP2015/056767
(87) International publication number: WO 2015/170506

(57) **Abstract**

A metal-organic framework (MOF) having the function of controlling the release of guests is provided in which reversible release control is possible and release control can be rapidly performed and which is capable of accommodating the release of various guest molecules. The metal-organic framework comprises both an organic ligand having two or more functional groups (coordinating functional groups) capable of coordinating to a metal atom and metal ions that combine with the coordinating functional groups of the organic ligand, and has a structure in which one metal ion has combined with two or more of the coordinating functional groups to connect multiple molecules of the organic ligand. The metal-organic framework/stimulus-responsive polymer composite was obtained by affixing a stimulus-responsive polymer to at least some of the surface of the metal-organic framework. The stimulus-responsive polymer may be affixed by bonding to the organic ligand.

## Description

### [Technical Field]

The present invention relates to a metal-organic framework/stimulus-responsive polymer composite capable of controlling release of guest molecules. More particularly, the present invention relates to a metal-organic framework comprised of organic ligands and metal ions linking the organic ligands, and a metal-organic framework/stimulus-responsive polymer composite in which a stimulus-responsive polymer being immobilized on at least a part of the surface of the metal-organic framework. The present invention further relates to a method for manufacturing a metal-organic framework/stimulus responsive polymer composite and to a method for manufacturing a metal-organic framework/stimulus responsive polymer composite containing a guest molecule.

### Cross-Reference to Related Applications

This application claims benefit of priority to Japanese Patent Application No. 2014-097006 filed on May 8, 2014, which is expressly incorporated herein by reference in its entirety.

### [Background Art]

Metal-organic frameworks (MOFs) are known as organic porous substances having organic materials in a base structure and nano-order pores (see Nonpatent Reference 1). A metal-organic framework is a framework in which organic ligands are accumulated by means of coordination bonds with metal ions.

Since MOFs are porous substances, they have the functions of trapping and releasing guest molecules, and their use as storage materials is anticipated. In particular, the releasing of guest molecules is an important function in use as a storage material. Known examples of reports relating to controlling the release of guest molecules by MOFs are: 1) those based on the addition of cations (Na⁺) to MOFs (Nonpatent Reference 2); 2) those in which an MOF is coated with silica and the decomposition of the MOF in response to pH is utilized (Nonpatent Reference 3); and 3) those utilizing drug molecules in the MOF structure and utilizing decomposition of the MOF (Nonpatent Reference 4).

### [Prior Technical References]

### [Nonpatent References]

Nonpatent Reference 1: O. M. Yaghi et al. Science, 2002, 295, 469.
Nonpatent Reference 2: J. An et al., J. Am. Chem. Soc. 2009, 131, 8376
Nonpatent Reference 3: W. Lin et al., J. Am. Chem. Soc. 2009, 131, 14261
Nonpatent Reference 4: S. R. Miller et al., Chem. Commun. 2010, 46, 4526

Nonpatent References 1 to 4 are expressly incorporated herein by reference in its entirety.

### [Summary of the Invention]

### [Problem To Be Solved by the Invention]

However, the release of guest molecules by the MOFs described in Nonpatent References 2 to 4 is achieved utilizing irreversible phenomena. MOFs affording reversible means of controlling the release are not known. In addition, a means permitting a rapid release control and a means permitting managing of the release of a variety of guest molecules are not known as well.

The present invention has for its object to provide an MOF having a controlled guest release function that enables to achieve the reversible release control, the rapid release control and the managing of the release of variety of guest molecules. A further object of the present invention is to provide a guest molecule-containing MOF employing an MOF having a function controlling the release of the guest.

### [Means of Solving the Problem]

The present invention is as set forth below.
[1] A metal-organic framework/stimulus-responsive polymer composite, comprising a metal-organic framework containing organic ligands having at least two functional groups capable of coordinating metal atoms (coordinating functional groups) and metal ions bonding with the coordinating functional groups of the organic ligands, and having a structure in which multiple organic ligands are connected such that one metal ion bonds with two or more coordinating functional groups, and a stimulus-responsive polymer being immobilized on at least a part of a surface of the metal-organic framework.
[2] The composite according to [1], wherein the stimulus-responsible polymer is immobilized on the metal-organic framework by bonding with the organic ligands.
[3] The composite according to [1] or [2], wherein the stimulus-responsive polymer is a temperature-responsive polymer, pH-responsive polymer, or light-responsive polymer.
[4] The composite according to [3], wherein the temperature-responsive polymer is of the lower critical solution temperature (LCST) type having an LCST or of the upper critical solution temperature (UCST) type having an UCST, the pH-responsive polymer is of a type in which a phase transition is induced by a change in pH, and the light-responsive polymer is of a type in which a phase transition is induced in response to light stimulation.
[5] The composite according to [4], wherein the LCST-type temperature-responsive polymer is a poly(N-alkylacrylamide), poly(N-alkylmethacrylamide), poly(N-vinylalkylacrylamide), poly(N-vinylmethacrylamide), polyvinyl alkyl ether, polyethylene glycol/polypropylene glycol block copolymer, amino group-comprising acrylic acid ester copolymer or methacrylic acid ester copolymer, polyethylene glycol derivative, acrylic acid ester polymer or methacrylic acid ester polymer comprising an oligoethylene glycol in a side chain, or a polyamino acid; and wherein the UCST-type temperature-responsive polymer is a polyethylene oxide, poly(vinylmethylether), poly(vinylalcohol), poly(hydroxyethyl methacrylate), poly(uracil acrylate), poly(methylacrylamide)/poly(N-acetylacrylamide) copolymer, poly(N-acryloylasparagine amide), poly(N-acryloylglutaminamide), poly(N-acryloylglucinamide), poly(N-methylacryloylasparagine amide), or poly(riboadenylate).
[6] The composite according to [3], wherein the pH-responsive polymer is a poly(N-vinylalkylacrylamide), poly(N-alkylacrylamide)/polymethacrylic acid copolymer, poly(N-alkylacrylamide)/polyacrylic acid copolymer, poly(2-ethoxyethylvinylether), polyisobutylvinylether, poly[6-(vinyloxy)hexanoic acid], poly[6-(2-vinyloxyethoxy)]hexanoic acid, or poly[4-(2-vinyloxyethoxy)benzoic acid.
[7] The composite according to [3], wherein the light-responsive polymer is a poly(N-vinylalkylacrylamide), poly(N-alkylacrylamide)/poly{6-[4-(4-pyridylazo)phenoxy]hexamethacrylate copolymer, poly(N-alkylacrylamide)/poly[n-(4-phenylazophenyl)acrylamide] copolymer, or poly{4-[2-(vinyloxy)ethoxy]azobenzene}/poly[2-(2-ethoxy)ethoxyethylvinylether] block copolymer.
[8] The composite according to any one of [5] to [7], wherein the poly(N-vinylalkylacrylamide) is poly-n-isopropylacrylamide (PNIPAM).
[9] The composite of any one of [1] to [8], wherein the modification rate of the surface of the metal-organic framework by the stimulus-responsive polymer falls within a range of greater than 0% and less than or equal to 25%, wherein the modification rate is a molar ratio of the numbers of reactive functional groups present in the organic ligand constituting the metal-organic framework and the stimulus-responsive polymer.
[10] The composite according to any one of [1] to [9], wherein the particle diameter of the metal-organic framework falls within a range of from 1 nm to 5 mm.
[11] A method for manufacturing a metal-organic framework/stimulus-responsive polymer composite, comprising the steps of:
   preparing a metal-organic framework comprising organic ligands that have at least two functional groups capable of coordinating metal atoms (coordinating functional groups) and reactive functional groups being reactive with the reactive functional groups present in a temperature-responsive polymer, and metal ions that bond with the coordinating functional groups of the organic ligands, and having a structure in which multiple organic ligands are connected such that one metal ion bonds with two or more coordinating functional groups; and
   subjecting the stimulus-responsive polymer comprising reactive functional groups and the metal-organic framework to conditions permitting reaction of the reactive functional groups present in the stimulus-responsive polymer with the reactive functional groups present in the organic ligands in the metal-organic framework to obtain a metal-organic framework/stimulus-responsive polymer in which the stimulus-responsive polymer is immobilized on at least a part of a surface of the metal-organic structure.
[12] The manufacturing method according to [11], in the form of a method for manufacturing the composite according to any one of [1] to [10].
[13] A method for manufacturing a guest molecule-containing metal-organic framework/temperature-responsive polymer composite including the capturing and sealing in of guest molecules into the metal-organic framework/temperature-responsive polymer composite according to any one of [1] to [10] (wherein the temperature-responsive polymer is of the lower critical solution temperature (LCST) type having a LCST), comprising the steps of:
   obtaining a metal-organic framework/temperature-responsive polymer composite in which guest molecules have been captured by immersing the metal-organic framework/temperature-responsive polymer composite in a solvent in which guest molecules have been dissolved or dispersed, at a temperature that is lower than the LCST of the temperature-responsive polymer; and
   exposing the metal-organic framework/temperature-responsive polymer composite in which the guest molecules have been captured to a temperature exceeding the LCST of the temperature-responsive polymer to seal the incorporated guest molecules within the metal-organic framework/temperature-responsive polymer composite and obtain a guest molecule-containing metal-organic framework/temperature-responsive polymer composite.
[14] A method for manufacturing a guest molecule-containing metal-organic framework/temperature-responsive polymer composite including the capturing and sealing in of guest molecules into the metal-organic framework/temperature-responsive polymer composite according to any one of [1] to [10] (wherein the temperature-responsive polymer is of the upper critical solution temperature (UCST) type having an UCST), comprising the steps of:
   obtaining a metal-organic framework/temperature-responsive polymer composite in which guest molecules have been captured by immersing the metal-organic framework/temperature-responsive polymer composite in a solvent in which guest molecules have been dissolved or dispersed, at a temperature that is higher than the UCST of the temperature-responsive polymer; and
   exposing the metal-organic framework/temperature-responsive polymer composite in which the guest molecules have been captured to a temperature less than or equal to the UCST of the temperature-responsive polymer to seal the incorporated guest molecules within the metal-organic framework/temperature-responsive polymer composite and obtain a guest molecule-containing metal-organic framework/temperature-responsive polymer composite.
[15] A method for manufacturing a guest molecule-containing metal-organic framework/pH-responsive polymer composite including the capturing and sealing in of guest molecules into the metal-organic framework/pH-responsive polymer composite according to any one of [1] to [10], comprising the steps of:
   obtaining a metal-organic framework/pH-responsive polymer composite in which guest molecules have been captured by immersing the metal-organic framework/pH-responsive polymer composite in a solvent in which guest molecules have been dissolved or dispersed in a pH range that dissolves the pH-responsive polymer; and
   exposing the metal-organic framework/pH-responsive polymer composite in which the guest molecules have been captured to a pH range at which the pH-responsive polymer is insoluble to seal the incorporated guest molecules within the metal-organic framework/pH-responsive polymer composite and obtain a guest molecule-containing metal-organic framework/pH-responsive polymer composite.
[16] A method for manufacturing a guest molecule-containing metal-organic framework/light-responsive polymer composite including the capturing and sealing in of guest molecules into the metal-organic framework/light-responsive polymer composite according to any one of [1] to [10], comprising the steps of:
   obtaining a metal-organic framework/light-responsive polymer composite in which guest molecules have been captured by immersing the metal-organic framework/light-responsive polymer composite in a solvent in which guest molecules have been dissolved or dispersed under ultraviolet radiation irradiation that dissolves the light-responsive polymer; and
   exposing the metal-organic framework/light-responsive polymer composite in which the guest molecules have been captured to visible light irradiation conditions under which the light-responsive polymer is insoluble to seal the incorporated guest molecules within the metal-organic framework/light-responsive polymer composite and obtain a guest molecule-containing metal-organic framework/light-responsive polymer composite.
[17] The method according to any one of [13] to [16], wherein the guest molecules are the active ingredient of a treatment drug, preventive drug, or test agent.

### [Effect of the Invention]

The present invention provides an MOF having a guest-molecule release control function that permits reversible release control, permits rapid release control, and can handle the release of a variety of guest molecules.

### [Brief Description of the Drawings]

Figure 1 shows SEM and TEM photographs of **UiO-NH₂-P1**.
Figure 2 shows the results of DLS measurement of **UiO-NH₂-P1** at room temperature.
Figure 3 shows an ATR-IR spectrum of **UiO-NH₂-BDC**, an ATR-IR spectrum of **P1**, and an ATR-IR spectrum of **UiO-NH₂-P1**.
Figure 4 shows a ¹H NMR spectrum of **P1,** a ¹H NMR spectrum of **UiO-NH₂-BDC**, and a ¹H NMR spectrum of **UiO-NH₂-P1** in a mixed solution of DMSO-*d*₆: HF aq. = 400 µL: 20 µL.
Figure 5 shows an XRPD pattern of UiO-66, an XRPD pattern of **UiO-NH₂-BDC**, and an XRPD pattern of **UiO-NH₂-P1**.
Figure 6 shows the release behavior of resorufin at 25°C and 40°C, the release behavior of caffeine at 25°C and 40°C, and the release behavior of procainamide at 25°C and 40°C based absorption spectra.
Figure 7 shows the release behavior of a guest molecule in the process of alternating the temperature between 20°C and 40°C at about 20 minute intervals using resorufin as a guest molecule.
Figure 8 tracks the release behavior of a guest molecule at 40°C in the measurement commencement stage and shows the release behavior of a guest molecule when the temperature was changed to 25°C at 60 minutes from the commencement of measurement using caffeine as guest molecule.
Figure 9 shows the guest molecule release behavior of **UiO-NH₂-BDC** at 40°C, the guest molecule release behavior of **UiO-NH₂-PNIPAM** at 25°C, and the guest molecule release behavior of **UiO-NH₂-PNIPAM** at 40°C using resorufin as guest molecule.
Figure 10 shows a TEM photograph of **UiO-NH₂-P2**.
Figure 11 shows the results of DLS measurement of **UiO-NH₂-P2** at room temperature.
Figure 12 shows an ATR-IR spectrum of **UiO-NH₂-BDC**, an ATR-IR spectrum of **P2,** and an ATR-IR spectrum of **UiO-NH₂-P2**.
Figure 13 shows a ¹H NMR spectrum of **P2,** a ¹H NMR spectrum of **UiO-NH₂-BDC**, and a ¹H NMR spectrum of **UiO-NH₂-P2** in a mixed solution of DMSO-*d*₆: HF aq. = 400 µL: 20 µL.
Figure 14 shows an XRPD pattern of UiO-66, an XRPD pattern of **UiO-NH₂-BDC**, and an XRPD pattern of **UiO-NH₂-P2**.
Figure 15 shows the release behavior of procainamide in pH buffer solutions (pH 6.86 or pH 4.01) in absorption spectra.
Figure 16 shows the guest molecule release behavior when the pH was alternated between greater than or equal to 7.3 and less than or equal to 4.4 at intervals of about 20 minutes using procainamide as guest molecule.
Figure 17 shows a TEM photograph of **UiO-NH₂-P3.**
Figure 18 shows an ATR-IR spectrum of **UiO-NH₂-BDC**, an ATR-IR spectrum of **P3,** and an ATR-IR spectrum of **UiO-NH₂-P3**.
Figure 19 shows a ¹H NMR spectrum of **P3,** a ¹H NMR spectrum of **UiO-NH₂-BDC**, and a ¹H NMR spectrum of **UiO-NH₂-P3** in a mixed solution of DMSO-*d*₆: HF aq. = 400 µL: 20 µL.
Figure 20 shows an XRPD pattern of UiO-66, an XRPD pattern of **UiO-NH₂-BDC**, and an XRPD pattern of **UiO-NH₂-P3**.
Figure 21 shows the release behavior of resorufin in visible light and UV radiation based on absorption spectra.
Figure 22 shows guest molecule release behavior with alternating irradiation with visible light and ultraviolet radiation at about 20 minute intervals using resorufin as the guest molecule.
Figure 23 shows the mechanism by which a pH-responsive polymer exhibits responsiveness.
Figures 24 A-C show the reaction schemas of Examples 1 to 3, respectively.

### [Modes of Carrying Out the Invention]

### [The metal-organic framework/stimulus-responsive polymer composite]

The present invention relates to a metal-organic framework/stimulus-responsive polymer composite, comprising a metal-organic framework containing organic ligands having at least two functional groups capable of coordinating metal atoms (coordinating functional groups) and metal ions bonding with the coordinating functional groups of the organic ligands, and having a structure in which multiple organic ligands are connected such that one metal ion bonds with two or more coordinating functional groups, with a stimulus-responsive polymer being immobilized on at least a part of the surface of the metal-organic framework. The stimulus-responsive polymer is desirably immobilized by bonding with the organic ligands.

The metal-organic framework is a substance that is obtained by solvothermally reacting the organic ligands and the metal ions. A description of the basic structure thereof is given, for example, in WO 2012/120905. The description of WO 2012/120905 is hereby incorporated in its entirety by reference.

### < The organic ligands >

The organic ligands have a molecular structure that is referred to as a "rigid molecule". The term "rigid molecule" is a molecule in which rotation and bending within the molecule are restricted. Examples are cyclic molecules, and aromatic rings or rod-shaped molecules connecting aromatic rings. Cyclodextrin is an example of a cyclic molecule. Examples of aromatic rings are phenylene, naphthalene, anthracenylene, pentacene, porphyrin, carborane, thiophene, pyridine, and fullerene (such as C60). Examples of rigid molecules are molecules containing one of these aromatic rings and molecules in which two or more aromatic rings are connected. The rigid molecule is desirably a molecule comprising one phenylene structure, a molecule having a diphenylene structure in which two phenylenes are connected, or a molecule having a terphenylene structure in which three phenylene groups are connected.

The organic ligand is a compound in which two or more functional groups capable of coordinating metal ions (coordinating functional groups) are present. Examples of coordinating functional groups are carboxyl groups, pyridinyl groups, cyano groups, amino groups, sulfonyl groups, porphyrinyl groups, acetyl acetonate groups, hydroxyl groups, Schiff bases, and amino acid residues. Desirable coordinating functional groups are carboxyl groups, pyridyl groups, cyano groups, amino groups, and sulfonyl groups. Carboxyl groups are preferred. The presence of a coordinating functional group in the form of a carboxyl group permits the formation of strong coordination bonds with metal ions. Desirably, each organic ligand can have two or more coordinating functional groups at any positions of the organic ligands. The coordinating functional groups can be present at the ends of the organic ligand, which is desirable from the perspective of obtaining a metal-organic framework the structure of which can be readily controlled and which affords relatively large pores.

In the present invention, in order for immobilization of the stimulus-responsive polymer, the organic ligand desirably has a reactive functional group reactive with the reactive functional group present on the stimulus-responsive polymer. One or two or more of such reactive functional groups can be incorporated into a single organic ligand. The type of reactive functional group can be suitably selected based on the type of reactive functional group present on the stimulus-responsive polymer. Examples are amino groups, azide groups, analogs thereof, double bonds, triple bonds, isocyanate groups, hydroxyl groups, thiol groups, and aldehyde groups. For example, when the reactive functional group that is present on the stimulus-responsive polymer is a succinimide group or isocyanate group, the reactive functional group can be an amino group. Alternatively, when the reactive functional group that is present on the stimulus-responsive polymer is an alkynyl group, the reactive functional group can be an azide group.

In formation of the metal-organic framework, a mixed system of organic ligands that do not comprise reactive functional groups and organic ligands that comprises reactive functional groups can be used. Alternatively, by using organic ligands having different types of reactive functional groups can be employed, temperature-responsive polymers of differing types having reactive functional groups of differing types with different physical properties can coexist on a single metal-organic framework.

The following compounds are included in examples of desirable organic ligands. These compounds can be obtained, for example, by referring to the description of O. M. Yaghi et al., Nature, Vol. 423, 12 June 2003, pp. 705-714 and H. Deng et al., Science, 336, 1018 (2012). The descriptions given in these documents are hereby incorporated in their entirety by reference.

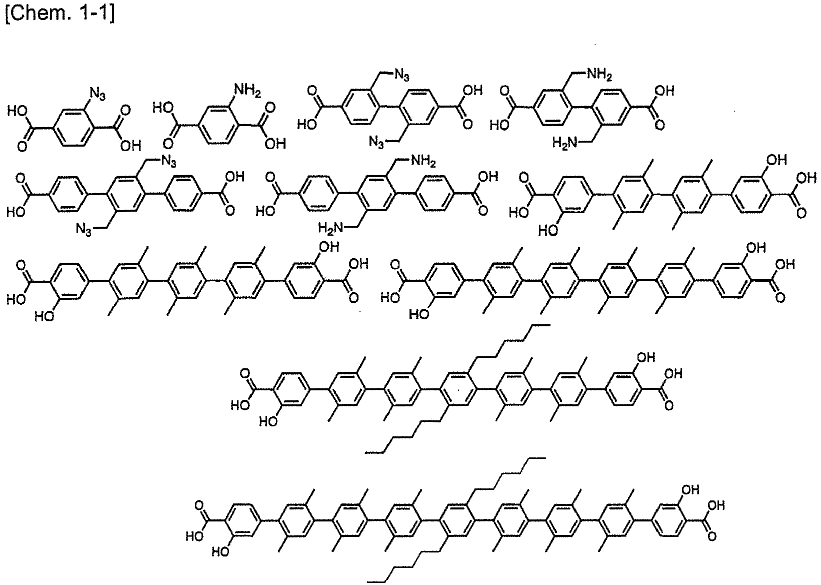

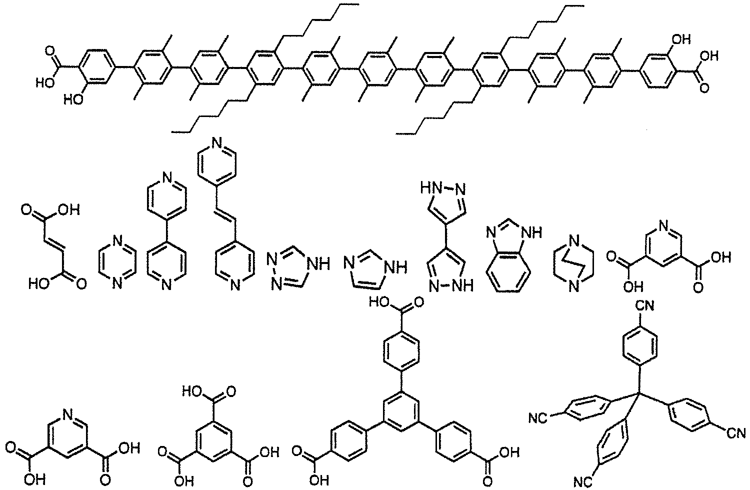

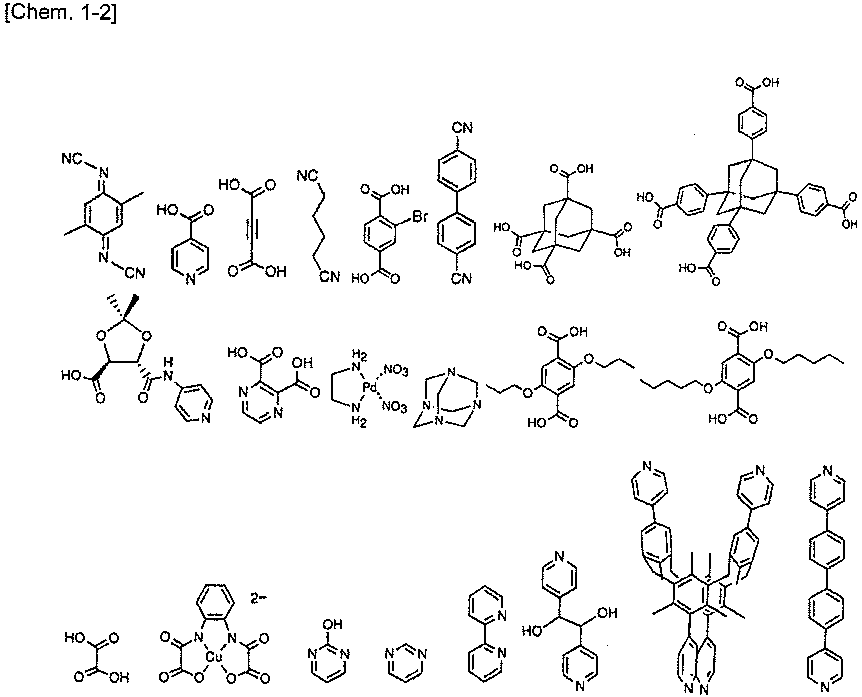

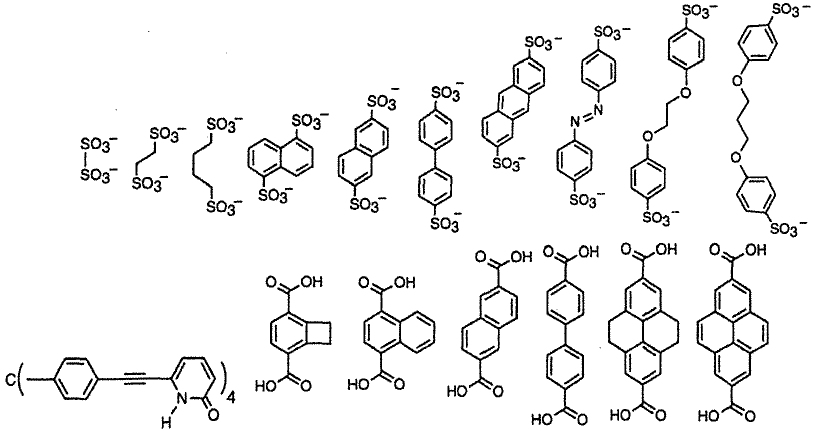

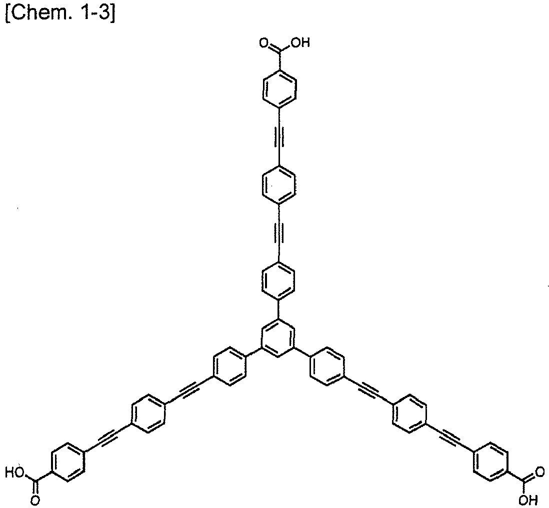

### < The metal ions >

By way of example, the metal ions can be metal ions of actinide elements or lanthanide elements in the form of metal elements of groups 1 through 16 of the Periodical Table of the Elements. Specific examples of metal ions are Li⁺, Na⁺, K⁺, Rb⁺, Be²⁺, Mg²⁺, Ca²⁺, Sr²⁺, Ba²⁺, SC³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺, Bi⁺, and combinations thereof. An example of a desirable metal ion is Zr⁴⁺.

### < The metal-organic framework >

The metal-organic framework can be manufactured by, for example, a solvothermal reaction of the organic ligands and metal ions by the usual methods (such as S. M. Cohen eta l., Chem. Commun., 2010, 46, 7700; V. Guillerm et al., Chem. Commun., 2010, 46, 767; N. Stock et al., Chem. Rev. 2012, 112, 933-969; and O. M. Yaghi et al., Science, 2002, 295, 469 (the entire contents of which are hereby incorporated by reference). The solvothermal reaction is normally conducted in the presence of an acid or a base at from room temperature to an elevated temperature (300°C). Solvent and starting materials are introduced under atmospheric pressure or into a pressurized vessel, the temperature is raised to above the boiling point, and the reaction is conducted under conditions where the pressure within the vessel is greater than or equal to atmospheric pressure. In addition to water, N,N-diethylformamide (DEF) or N,N-dimethylformamide (DMF) can be employed as the solvent in the solvothermal reaction since they decompose at elevated temperature, gradually producing amine bases. The conditions of the solvothermal reaction can be suitably set.

The metal-organic framework desirably has crystalline properties. From the perspective of facilitating the manufacturing of a metal-organic framework having crystalline properties or being highly crystalline, an inhibitor capable of controlling the surface energy during crystal growth, such as an organic acid such as acetic acid, or benzoic acid, can be employed. However, a metal-organic framework that has crystalline properties or is highly crystalline can be manufactured without use of an inhibitor. The presence of crystalline properties can be confirmed by observing the diffraction pattern in X-ray diffraction.

The metal-organic framework is a porous substance having nano-order pores. The size of the pores is controlled by means of the length of the organic ligands in the lengthwise direction (the distance between coordinating functional groups present on the organic ligands). Specifically, the pore diameter, for example, falls within a range of 0.1 to 10 nm, a range of 0.1 to 5 nm, a range of 0.1 to 3 nm, a range of 0.1 to 2 nm, or a range of 0.1 to 1 nm. Taking into account the size of the guest molecules trapped in the composite of the present invention, suitable adjustment can be achieved through selection of the organic ligands.

The metal-organic framework is a powder or particulate material. The diameter of the particles, for example, falls within a range of from 1 nm to 5 mm, within a range of 10 nm to 4 mm, within a range of 30 nm to 3 mm, or within a range of 50 nm to 2 mm. The particle diameter of the metal-organic framework can be controlled over a broad range through selection of the synthesis conditions. A small size in the form of nano-order particles can be achieved, while a large size in the form of milli-order particles can be achieved.

### < The metal-organic framework/stimulus-responsive polymer composite >

The metal-organic framework/stimulus-responsive polymer composite of the present invention is a metal-organic framework/stimulus-responsive polymer composite in which a stimulus-responsive polymer is immobilized on at least a part of the surface of the metal-organic framework.

The temperature-responsive polymer is generally a polymer the solubility in water of which changes dramatically with changes in temperature. Such polymers come in types: the LCST type, which is soluble in water at low temperatures but becomes insoluble when the temperature is raised to a certain level (the lower critical solution temperature: LCST) and the UCST type, which is soluble in water at elevated temperatures but becomes insoluble when the temperature is increased to a certain level (the upper critical solution temperature: UCST). The average molecular weight of the temperature-responsive polymer is not specifically limited. By way of example, the number average molecular weight falls within a range of 1,000 to 30,000, desirably within a range of 1,000 to 20,000, preferably within a range of 1,000 to 10,000, more preferably within a range of 1,000 to 8,000, and optimally, within a range of 1,000 to 5,000. However, no restriction to within these ranges is intended. The average molecular weight of the temperature-responsive polymer can be measured, for example, by GPC (chloroform).

Examples of known LCST-type temperature-responsive polymers are poly(N-alkylacrylamide), poly(N-alkylmethacrylamide), poly(N-vinylalkylacrylamide), poly(N-vinylmethacrylamide), polyvinyl alkyl ether, and polyethylene glycol/polypropylene glycol block copolymers. Examples of other known LCST-type temperature-responsive polymers are methacrylic acid ester polymers and acrylic acid ester polymers having amino groups, polyethylene glycol derivatives, methacrylic acid ester polymers and acrylic acid ester polymers having oligoethylene glycol on side chains, and polyamino acids.

Examples of known UCST-type temperature responsive polymers are polyethylene oxide, poly(vinylmethylether), poly(vinylalcohol), poly(hydroxyethyl methacrylate), poly(uracil acrylate), poly(methylacrylamide)/poly(N-acetylacrylamide) copolymer, poly(N-acryloylasparagine amide), poly(N-acryloylglutaminamide), poly(N-acryloylglucinamide), poly(N-methylacryloylasparginamide, and poly(riboadenylate).

In the present invention, the temperature-responsive polymer is not specifically limited. For example, it can be a poly(N-alkylacrylamide) in the form of a polyacrylamide selected from the group consisting of poly-n-isopropylacrylamide (PNIPAM), poly-n-n-dipropylmethacrylamide, and poly-n-n-diethylacrylamide. Poly-n-isopropylacrylamide (PNIPAM) has an LCST at 32°C, near body temperature, and is thus desirable as a DDS material.

Reference can be made to the following documents in regard to the temperature-responsive polymer. The entire contents thereof are hereby incorporated by reference.

### [Reference documents for temperature-responsive polymers]

- Wang, X.; Qiu, X.; Wu, C. Macromolecules 1998, 31, 2972.
- Aoshima, S.; Oda, H.; Kobayashi, E. Journal of Polymer Science Part A: Polymer Chemistry 1992, 11, 2407.
- Yoshimitsu, H.; Kanazawa, A.; Kanaoka, S.; Aoshima, S. Macromolecules, 2012, 45, 9427.

The entire contents thereof are hereby incorporated by reference.

pH-responsive polymers are polymers the solubility of which in solution (such as an aqueous solution or pH buffer) changes dramatically with changes in pH. They come in types: a type that dissolves in solution in the high pH range but becomes insoluble when the pH drops to a certain level, and a type that dissolves in solution in the low pH range but becomes insoluble when the pH rises to a certain level.

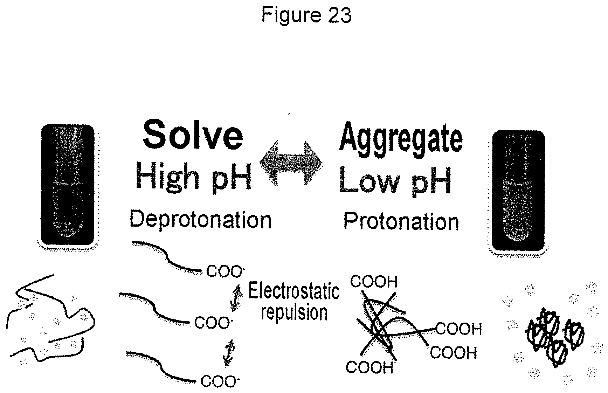

Figure 23 shows a schema of the mechanisms by which pH-responsive polymers exhibit responsiveness.

The average molecular weight of the pH-responsive polymer is not specifically limited. By way of example, the number average molecular weight falls within a range of 1,000 to 30,000, desirably within a range of 1,000 to 20,000, preferably within a range of 1,000 to 10,000, and more preferably, within a range of 1,000 to 8,000. However, limitation to within this range is not intended. The average molecular weight of the pH-responsive polymer can be measured, for example, by GPC (chloroform).

The pH-responsive polymer is not specifically limited. Known examples are: poly(N-alkylacrylamide)/polymethacrylic acid copolymers, poly(N-alkylacrylamide)/polyacrylic acid copolymers, poly(2-ethoxyethylvinylether), polyisobutylvinylether, and poly[6-(vinyloxy)hexanoic acid].

Examples of pH-responsive polymers are poly(N-alkylacrylamide) polymers in the form of poly-n-isopropylacrylamide (PNIPAM)/polyacrylic acid copolymers. Since the pH range at which a poly-n-isopropylacrylamide (PNIPAM)/polyacrylic acid copolymer dissolves can be varied by means of its copolymerization ratio, it is desirable as a DDS material. Examples of polyvinyl ether type pH-responsive polymers are poly[6-(2-vinyloxyethoxy)]hexanoic acid and poly[4-(2-vinyloxyethoxy)]benzoic acid.

Reference can be made to the following documents regarding pH-responsive polymers. The entire contents of these documents are hereby incorporated by reference.

### [pH-responsive polymer reference documents]

- Gu, J.; Xia, F.; Wu, Y.; Qu, X.; Yang, Z.; Jiang, L. Journal of Controlled Release 2007, 117, 396.
- Hoare, T.; Pelton, R. Macromolecules 2004, 37, 2544.
- Mohan, Y. M.; Premkumar, T.; Joseph, D. K.; Geckeler, K. E. Reactive & Functional Polymers 2007, 67, 844.
- Yoo, M. K.; Sung, Y. K.; Lee, Y. M. Cho, C. S. Polymer 2000, 41, 5713.
- Oda, Y.; Tsujino, T.; Kanaoka, S.; Aoshima, S. Journal of Polymer Science Part A: Polymer Chemistry 2012, 50, 2993.

The entire contents of these documents are hereby incorporated by reference.

Light-responsive polymers are generally polymers the solubility of which, in solvent, changes dramatically when irradiated with light. They come in types: a type that dissolves in solvent when irradiated with visible light, but becomes insoluble when irradiated with ultraviolet radiation, and a type that dissolves when irradiated with ultraviolet radiation but becomes insoluble when irradiated with visible light. The mechanism by which light-responsive polymers exhibit responsiveness is a change in the polarity of the polymer due to a *cis* (soluble)-*trans* (aggregation) isomerization reaction of azobenzen and azopyridine.

The average molecular weight of the light-responsive polymer is not specifically limited. By way of example, the number average molecular weight falls within a range of 1,000 to 30,000, desirably within a range of 1,000 to 20,000, preferably within a range of 1,000 to 10,000, and more preferably, within a range of 1,000 to 8,000. However, limitation to within this range is not intended. The average molecular weight of the light-responsive polymer can be measured, for example, by GPC (chloroform).

Examples of light-responsive polymers are poly(N-alkylacrylamide)/poly{6-[4-(4-pyridylazo)phenoxy]hexamethacrylate copolymers, poly(N-alkylacrylamide)/poly[n-(4-phenylazophenyl)acrylamide]copolymers, and poly{4-[2-(vinyloxy)ethoxy]azobenzene}/poly[2-(2-ethoxy)ethoxyethylvinylether]block copolymers.

In the present invention, the light-responsive polymer is not specifically limited. An example is a poly(N-alkylacrylamide) in the form of a poly-n-isopropylacrylamide (PNIPAM)/poly{6-[4-(-pyridylazo)phenoxy]hexamethacrylate copolymer. Poly-n-isopropylacrylamide (PNIPAM)/poly{6-[4-(-pyridylazo)phenoxy]hexamethacrylate copolymer is desirable as a DDS material because it is possible to change the polar solvent in which it is soluble by means of the copolymerization ratio.

Reference can be made to the following documents with regard to the light-responsive polymer. The entire contents of these documents are hereby incorporated by reference.

### [Light-responsive polymer reference documents]

- Irie, M.; Kungwatchakun, D. Proc. Japan Acad. Ser. B 1992, 68, 127.
- Han, K.; Su, W.; Zhong, M.; Yan, Q.; Luo, Y.; Zhang, Q.; Li, Y. Macromol. Rapid Commun. 2008, 29, 1866.
- Shen, G.; Xue, G.; Cai, J.; Zou, G. Li, Y.; Zhong, M.; Zhang, Q. Soft Matter 2012, 8, 9127.
- Yoshida, T.; Kanaoka, S. Aoshima, S. Journal of Polymer Science Part A: Polymer Chemistry 2005, 43, 5337.
- Cui, L.; Zhao, Y. Chem. Mater. 2004, 16, 2076.

The entire contents of these documents are hereby incorporated by reference.

Polymers having both pH responsiveness and temperature responsiveness based on the constituent components of the polymer are also known. The present invention includes metal-organic framework/stimulus-responsive polymer composites in which such dual stimulus-responsive polymers are employed as the stimulus-responsive polymer.

In the metal-organic framework/stimulus-responsive polymer composite of the present invention, the stimulus-responsive polymer is immobilized on at least a part of the surface of the metal-organic framework. The stimulus-responsive polymer is desirably immobilized by bonding the organic ligands constituting to the metal-organic framework to the stimulus-responsive polymer. Specifically, bonding is desirably achieved by reacting the reactive functional groups present on the organic ligand with the reactive functional groups present on the stimulus-responsive polymer. Although the reactive functional groups present on the organic ligand and the reactive functional groups present on the stimulus-responsive polymer are not limited, the bond formed by reacting the groups can be an amide bond, 1,2,3-triazole bond, disulfide bond, hydrazone bond, or thioether bond. Examples of the reactive functional groups present on the organic ligand and the reactive functional groups present on the stimulus-responsive polymer that form these bonds are given in the following table. Functional Group 1 is an example of a reactive functional group present on the organic ligand and Functional Group 2 is an example of a reactive functional group present on the stimulus-responsive polymer. The two can also be interchanged.

**[Table 1]**

| Functional Group 1 | Functional Group 2 | Bond formed |
|---|---|---|
| Amino group | Succinate ester group | Amide |
| Azide group | Alkynyl group | 1,2,3-Triazole |
| Thiol group | Mercapto group | Disulfide |
| Aldehyde group | Hydrazide group | Hydrazone |
| Thiol group | Maleimide group | Thioether |
| Amino group | Isocyanate group | Urea |

### [Method for manufacturing a metal-organic framework/stimulus-responsive polymer composite]

The method for manufacturing a metal-organic framework/stimulus-responsive polymer composite of the present invention comprises (1) a step of preparing the following metal-organic framework and (2) a step of obtaining a metal-organic framework/stimulus-responsive polymer composite.

Step (1) of preparing a metal-organic framework is a step of preparing a metal-organic framework that comprises organic ligands containing two or more functional groups capable of coordinating with metal atoms (coordinating functional groups) and having reactive functional groups that react to reactive functional groups present on the stimulus-responsive polymer and metal ions bonding with the coordinating functional groups of the organic ligands, and that has a structure in which multiple organic ligands are linked by the bonding of each metal ion to two or more coordinating functional groups. The details regarding this step are as set forth above for the method of preparing the metal-organic framework.

Step (2) of obtaining a metal-organic framework/stimulus-responsive polymer composite is a step of obtaining a metal-organic framework/stimulus-responsive polymer composite by subjecting the metal-organic framework and the stimulus-responsive polymer having reactive functional groups to conditions under which the reactive functional groups present on the organic ligands in the metal-organic framework react with the reactive functional groups present on the stimulus-responsive polymer, thereby immobilizing the stimulus-responsive polymer on at least a part of the surface of the metal-organic framework.

The reaction of the reactive functional groups present on the organic ligand with reactive functional groups present on other molecules can be conducted according to the method described by K. Sada et al., CrystEngComm, 2012, 14, 4137. Suitable adjustment is possible based on the type of reactive functional groups present on the organic ligands and the type of reactive functional groups present on the stimulus-responsive polymer.

For example, for organic ligands having responsive functional groups, a stimulus-responsive polymer having reactive functional groups can be mixed in an organic solvent in a molar ratio of the reactive functional groups of the stimulus-responsive polymer to the reactive functional groups of the organic ligands falling within a range of 1:0.1 to 10, desirably falling within a range of 1:0.5 to 5, and preferably falling within a range of 1:1 to 2 and then left standing for a prescribed period to obtain a metal-organic framework/stimulus-responsive polymer composite. The organic solvent is not specifically limited beyond that it dissolves the stimulus-responsive polymer and disperses the organic ligands. For example, chloroform can be employed. The (reaction) temperature during standing is suitably selected from within a range of from room temperature (such as 20°C) to the boiling point of the organic solvent taking into account the type of organic ligand and stimulus-responsive polymer. However, it is also possible to implement the reaction at a temperature exceeding the boiling point of the organic solvent by using an autoclave, for example, depending on the type of organic ligand and stimulus-responsive polymer. The (reaction) standing time can be suitably determined, for example, within a range of from 1 minute to 100 hours taking into account the quantity (yield) of metal-organic framework/stimulus-responsive polymer composite produced. However, no limitation to this range is intended.

Once the reaction has ended, the solid and liquid are separated from the organic solvent by centrifugation, for example, and washed as needed to obtain a metal-organic framework/stimulus-responsive polymer composite.

The mass ratio of the metal-organic framework and the stimulus-responsive polymer that is immobilized on the metal-organic framework is not specifically limited. Although depending on the type and molecular weight of the stimulus-responsive polymer, the size of the stimulus-responsive polymer is generally greater than the size of the pores of the metal-organic framework so that the stimulus-responsive polymer does not enter the pores of the metal-organic framework but is immobilized on the surface of the metal-organic framework. In that case, the mass ratio of the stimulus-responsive polymer that is immobilized on the metal-organic framework is determined primarily by the type (particularly the number of reactive functional groups present on the organic ligands constituting the metal-organic framework) and surface area of the metal-organic framework, and the molecular weight of the stimulus-responsive polymer. In terms of the performance of the composite of the present invention, from the perspectives of confining within the metal-organic framework the guest molecules that have been trapped by the metal-organic framework and effectively releasing them, the type of anticipated guest molecule and the type of stimulus-responsive polymer can be taken into account to suitably determine the mass ratio of the stimulus-responsive polymer that is immobilized on the metal-organic framework. The molar ratio of the stimulus-responsive polymer immobilized on the metal-organic framework and the number of reactive functional groups present on the organic ligands constituting the metal-organic framework (referred to as the "modification rate" of the stimulus-responsive polymer) is, for example, greater than 0%, and can fall within a range of less than or equal to 25%. The modification rate desirable falls within a range of 1 to 20%, preferably falls within a range of 2 to 15%, and more preferably, falls within a range of 3 to 15%.

The modification rate can be calculated by obtaining the amount of organic ligands with which the stimulus-responsive polymer has actually been modified from the integral ratio of the peaks derived from the organic ligands in the metal-organic framework before and after modification based on the ¹H NMR of the metal-organic framework before modification with the stimulus-responsive polymer and the ¹H NMR spectrum of the metal-organic framework after modification with the stimulus-responsive polymer. The quantity of stimulus-responsive polymer needed to cover the entire outermost surface of the metal-organic framework (referred to as the outermost surface coverage modification rate) can be estimated as follows. An estimate can be obtained by assuming that the MOF is a regular octahedron, the length of one side of the MOF is 200 nm, and the thickness of the lattice structure of the outermost surface is 14 A. The actual modification rate will sometimes be greater than the outermost surface coverage modification rate. That is because the metal-organic framework is porous and the reaction between the reactive functional groups present on the organic ligands present internally and the stimulus-responsive polymer readily occurs. For example, in the case of **UiO-NH₂-P1** given in the examples, the outermost surface coverage modification rate is estimated at 5.14%, while the modification rate of P1 portions determined from the ¹H NMR spectrum is about 11%.

### [Method for manufacturing a guest molecule-containing metal-organic framework/stimulus-responsive polymer composite]

The present invention also covers a method for manufacturing a guest molecule-containing metal-organic framework/stimulus-responsive polymer composite. This method comprises incorporating guest molecules into the metal-organic framework/stimulus-responsive polymer composite of the present invention to obtain a metal-organic framework/stimulus-responsive polymer composite in which guest molecules have been trapped and sealed (clathrated).

In the case of an LCST type stimulus-responsive polymer having a lower critical solution temperature (LCST), trapping of the guest molecule is implemented by immersing the metal-organic framework/temperature-responsive polymer composite in a solvent in which the guest molecules have been dissolved or dispersed at a temperature lower than the LCST of the temperature-responsive polymer. The LCST of the temperature-responsive polymer is an inherent characteristic (temperature) of the temperature-responsive polymer. The temperatures at which the guest molecules are trapped and released can be set by selecting the type of temperature-responsive polymer in consideration of the temperature range at which the guest molecules are to be trapped and released. At temperatures below the LCST of the temperature-responsive polymer, the temperature-responsive polymer dissolves in the solvent in which the guest molecules have been dissolved or dispersed. Since they are immobilized on the metal-organic framework, a state of affinity is created in the solvent. The guest molecules can move from the solvent toward the metal-organic framework between the temperature-responsive polymer chains in a free state. As a result, the guest molecules are trapped by the metal-organic framework.

In the case of a UCST type stimulus-responsive polymer having an upper critical solution temperature (UCST), trapping of the guest molecule is implemented by immersing the metal-organic framework/temperature responsive polymer composite in solvent in which the guest molecules have been dissolved or dispersed at a temperature exceeding the UCST of the temperature-responsive polymer. The UCST of the temperature-responsive polymer is an inherent characteristic (temperature) of the temperature-responsive polymer. The temperatures at which the guest molecules are trapped and released can be set by selecting the type of temperature-responsive polymer in consideration of the temperature range at which the guest molecules are to be trapped and released. At temperatures above the UCST of the temperature-responsive polymer, the temperature-responsive polymer dissolves in the solvent in which the guest molecules have been dissolved or dispersed. Since they are immobilized on the metal-organic framework, a state of affinity is created in the solvent. A "state of affinity" means a state in which the polymer with which the surface of the metal-organic framework has been modified forms hydrogen bonds with the water molecules in the solvent, and the polymer chains extend. The guest molecules can move from the solvent toward the metal-organic framework between the temperature-responsive polymer chains in a free state. As a result, the guest molecules are trapped by the metal-organic framework.

In the case of a stimulus-responsive polymer that responds to a change in pH by inducing a phase transition, trapping of the guest molecule is implemented by immersing the metal-organic framework/temperature responsive polymer composite in solvent in which the guest molecules have been dissolved or dispersed at a pH range that dissolves the pH-responsive polymer. The dissolution range of the pH-responsive polymer is an inherent characteristic (pH) of a pH-responsive polymer. The pH ranges at which the guest molecules are trapped and released can be set by selecting the type of pH-responsive polymer in consideration of the pH ranges at which the guest molecules are to be trapped and released. Within the dissolution range of the pH-responsive polymer, the pH-responsive polymer assumes a state in which the pH-responsive polymer dissolves into the solvent in which the guest molecules have been dissolved or dispersed, creating a state of affinity in the solvent because the pH-responsive polymer is immobilized on the metal-organic framework. The guest molecules can move from the solvent toward the metal-organic framework between the pH-responsive polymer chains in a free state. As a result, the guest molecules are trapped by the metal-organic framework.

In the case of a stimulus-responsive polymer that induces a phase transition in response to light stimulus, trapping of the guest molecule is implemented by immersing the metal-organic framework/light-responsive polymer composite in a solvent in which the gest molecules have been dissolved or dispersed with the radiation of light having a wavelength that dissolves the light-responsive polymer. The dissolution range of the light-responsive polymer is an inherent characteristic (wavelength of irradiated light) of a light-responsive polymer. The wavelength of the irradiated light that traps and releases the guest molecules can be set by selecting the type of light-responsive polymer in consideration of the dissolution range at which the guest molecules are to be trapped and released. Within the dissolution range of the light-responsive polymer, the light-responsive polymer assumes a state in which the light-responsive polymer dissolves into the solvent in which the guest molecules have been dissolved or dispersed, creating a state of affinity in the solvent because the light-responsive polymer has been immobilized on the metal-organic framework. The guest molecules can move from the solvent toward the metal-organic framework between the light-responsive polymer chains in a free state. As a result, the guest molecules are trapped by the metal-organic framework.

The quantity of guest molecules trapped by the metal-organic framework/stimulus-responsive polymer composite and the rate at which they are trapped vary with the state of the guest molecules in the solvent (a state of dissolution or dispersion) and the concentration of the guest molecules and temperature (free state of the stimulus-responsive polymer). Thus, suitable control can be achieved by adjusting these factors. The solvent in which the guest molecules are dissolved or dispersed can be suitably determined based on the type of guest molecule. Examples are water, organic solvents, and mixed systems of water and organic solvents. For example, when employing an organic solvent, methanol, ethanol, propanol, toluene, hexane, N,N-dimethylformamide (DMF), N,N-diethylformamide (DEF), chloroform, dichloromethane, diethylether, dimethylsulfoxide (DMSO), tetrahydrofuran (THF), acetonitrile, 1,4-dioxane, and the like can be employed.

The type of guest molecule is not limited. Examples are the active ingredients of treatment drugs, preventive drugs, and test agents. More specific examples are the active ingredients of cancer or malignant tumor treatment drugs, preventive drugs, and test agents. Examples are given below. However, these are just examples and are not intended as limits.

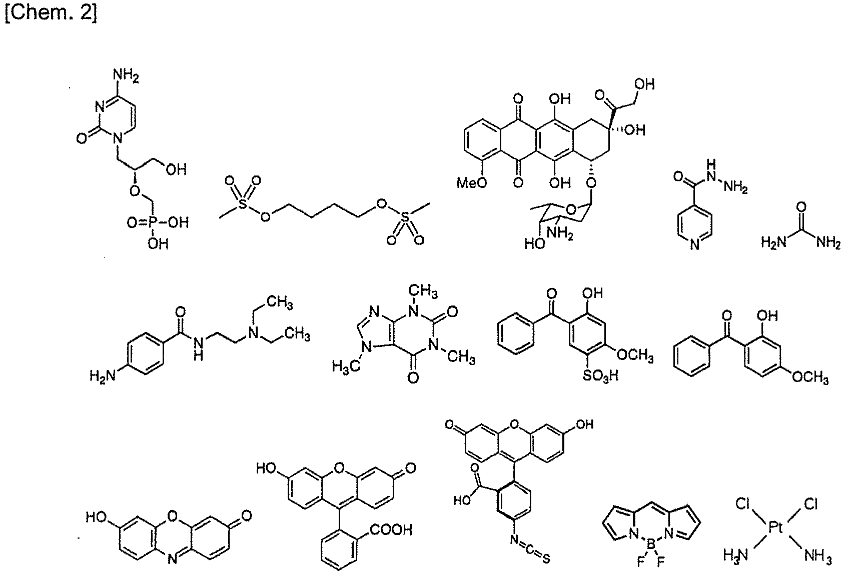

In case the stimulus-responsive polymer is the LCST type, the guest drug is trapped for a prescribed period and the metal-organic framework/stimulus-responsive polymer composite in which the guest molecule has been trapped is exposed to a temperature exceeding the LCST of the temperature-responsive polymer, the temperature-responsive polymer is caused to aggregate, and the guest molecules that have been trapped are sealed within the metal-organic framework/temperature-responsive polymer composite. Specifically, the guest molecules are trapped for a prescribed period, after which the solvent containing the metal-organic framework/temperature-responsive polymer composite and the guest molecules is heated to a temperature exceeding the LCST of the temperature-responsive polymer. This heating is desirably effected relatively quickly from the perspective of better ensuring that the guest molecules are sealed within the composite.

In case the stimulus-responsive polymer is the UCST type, the guest molecule is trapped for a prescribed period and the metal-organic framework/stimulus-responsive polymer composite in which the guest molecule has been trapped is exposed to a temperature below the UCST of the temperature-responsive polymer, the temperature-responsive polymer is caused to aggregate, and the guest molecules that have been trapped are sealed within the metal-organic framework/temperature-responsive polymer composite. Specifically, the guest molecules are trapped for a prescribed period, after which the solvent containing the metal-organic framework/temperature-responsive polymer composite and the guest molecules is cooled to a temperature less than or equal to the UCST of the temperature-responsive polymer. This cooling is desirably effected relatively quickly from the perspective of better ensuring that the guest molecules are sealed within the composite.

When the guest molecule-containing metal-organic framework/temperature-responsive polymer composite that has been prepared by the above method is once again exposed in a suitable solvent to a temperature below the lower critical solution temperature of the temperature-responsive polymer, the guest molecules are released by the metal-organic framework. That is, the temperature-responsive polymer assumes a free state in the solvent and the guest molecules go from a state of being sealed in the metal-organic framework to a state in which they can be released. The solvent in which the guest molecules will be released is not specifically limited. When employing the metal-organic framework/temperature-responsive polymer composite of the present invention in a drug dispensing system (DDS), the solvent can also be a body fluid (such as blood, lymph fluid, or saliva).

In case the stimulus-responsive polymer is the type inducing a phase change in response to a change in pH, the guest molecule is trapped for a prescribed period and the metal-organic framework/stimulus-responsive polymer composite in which the guest molecule has been trapped is exposed to a solvent in a pH range at which the temperature-responsive polymer is insoluble, the pH-responsive polymer is caused to aggregate, and the guest molecules that have been trapped are sealed within the metal-organic framework\temperature-responsive polymer composite. Specifically, the guest molecules are trapped for a prescribed period, after which the solvent containing the metal-organic framework/pH-responsive polymer composite and the guest molecules is immersed in a solvent at a pH range at which the pH-responsive polymer is insoluble. This operation is desirably effected relatively quickly from the perspective of better ensuring that the guest molecules are reliably sealed within the composite.

When the guest molecule-containing metal-organic framework/pH-responsive polymer composite that has been prepared by the above method is once again exposed to a solvent at a pH range at which the pH-responsive polymer dissolves, a state in which the guest molecules are released by the metal-organic framework is created. That is, the pH-responsive polymer assumes a free state in the solvent and the guest molecules go from a state of being sealed in the metal-organic framework to a state in which they can be released.

In case the stimulus-responsive polymer is the type inducing a phase change in response to stimulus with light, the guest molecule is trapped for a prescribed period and the metal-organic framework/stimulus-responsive polymer composite in which the guest molecule has been trapped is exposed within the visible light range at which the light-responsive polymer is insoluble, the light-responsive polymer is caused to aggregate, and the guest molecules that have been trapped are sealed within the metal-organic framework/light-responsive polymer composite. Specifically, the guest molecules are trapped for a prescribed period, after which the solvent containing the metal-organic framework/light-responsive polymer composite and the guest molecules is irradiated with visible light that does not dissolve the light-responsive polymer. This operation is desirably effected relatively quickly from the perspective of better ensuring that the guest molecules are reliably sealed within the composite.

When the guest molecule-containing metal-organic framework/light-responsive polymer composite that has been prepared by the above method is once again exposed to irradiation with ultraviolet radiation that dissolves the light-responsive polymer, a state in which the guest molecules are released by the metal-organic framework is created. That is, the light-responsive polymer assumes a free state in the solvent and the guest molecules go from a state of being sealed in the metal-organic framework to a state in which they can be released.

### [Examples]

The present invention will be described in greater detail based on examples. However, the examples are examples of the present invention. There is no intent to limit the present invention to the examples.

### Analysis devices

### (1) Scanning electron microscope (SEM) photography

- Device: JSM-7400, Japan Electronics (Ltd.)
- Acceleration voltage: 1.0 kV
- Sample processing: The samples are not processed with electrically conductive substances

### (2) Transmission electron microscope (TEM) photography

- Device: H-8000 Hitachi High Technologies (Ltd.)
- Operating voltage: 200 kV
- Sample processing: The samples are not processed with electrically conductive substances

### (3) ¹H-NMR spectrum

- Device: Advance 500 (500 MHz), made by Bruker Bio-Spin (Ltd.)

### (4) X-ray diffraction device (XRPD)

- Measurement device: D8 Advance, made by Bruker Bio-Spin (Ltd.)

### (5) Total reflection measurement method IR (ATR-IR)

- Measurement device: FTIR-4100 SK, Japan Electronics (Ltd.)

### (6) DLS

- Measurement device: Beckman-Coulter Delsa Nano HC

### Starting material

### MonoNH₂-BDC (BDC: Benzenedicarboxylic acid)

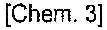

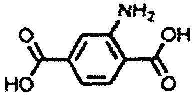

### Polymer P1

Number average molecular weight = 2,000 (catalog value)
Results of GPC (chloroform) measurement:
   Number average molecular weight = 2,000
   Weight average molecular weight = 2,100
   Molecular weight distribution = 1.05

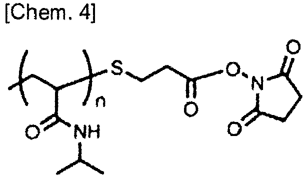

### Polymer P2

Number average molecular weight = 2,000 (catalog value)
Results of GPC (chloroform) measurement:
   Number average molecular weight = 4,100
   Weight average molecular weight = 4,200
   Molecular weight distribution = 1.02

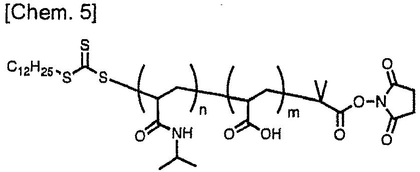

### Polymer P3

Number average molecular weight = 2,000 (catalog value)
Results of GPC (chloroform) measurement:
   Number average molecular weight = 4,630
   Weight average molecular weight = 4,150
   Molecular weight distribution = 1.12

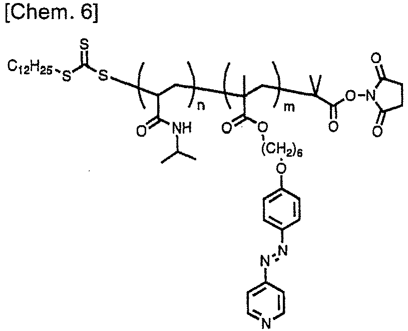

**[Table 2]**

| - Responsiveness and molecular weight of each polymer | | | |
|---|---|---|---|
| Individual polymer | Number average molecular weight | Weight average molecular weight | Molecular weight distribution (Mw/Mn) |
| P1 (temperature-responsive) | 2,000 | 2,100 | 1.05 |
| P2 (pH-responsive) | 4,100 | 4,200 | 1.02 |
| P3 (light-responsive) | 4,630 | 4,150 | 1.12 |

### Reference Example 1 Synthesis of MOF (UiO-NH₂-BDC) comprising amino groups

To an autoclave test tube were added **MonoNH²-BDC** (11 mg, 51 µmol) and ZrCl₄ (12 mg, 51 µmol). This was dissolved in DMF (600 mL). Subsequently, the solution was heated to 120°C and left standing for 24 hours. After standing, the solution was cooled to room temperature. The sample was recovered by centrifugal separation (2,000 rpm, 3 min) and washed with DMF and methanol to obtain regular decahedral yellow crystals (about 20 mg). The fact that the targeted **UiO-NH₂-BDC** had been obtained was confirmed by SEM and TEM observation, ATR-IR spectral measurement, and XRPD measurement.

### Example 1

### (1) Synthesis of MOF (UiO-NH₂-P1) having P1 moieties

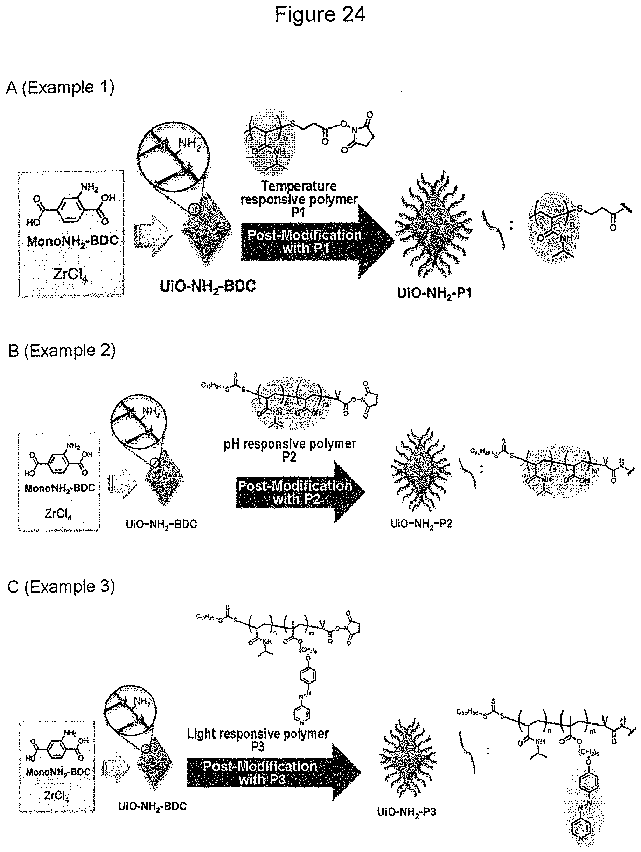

**UiO-NH₂-BDC** (60 mg, about 0.2 mmol: -NH₂) was added to a screw tube, 500 µL of a 0.1 M P1 (400 mg, 0.2 mmol) chloroform solution was added, and the mixture was left standing for 48 hours at 60°C. Subsequently, the MOF was recovered by centrifugal separation (2,000 rpm, 3 min) and washing was conducted with chloroform and methanol. This yielded about 50 mg of the targeted UiO-NH2-P1. The reaction schema is given in Figure 24A.

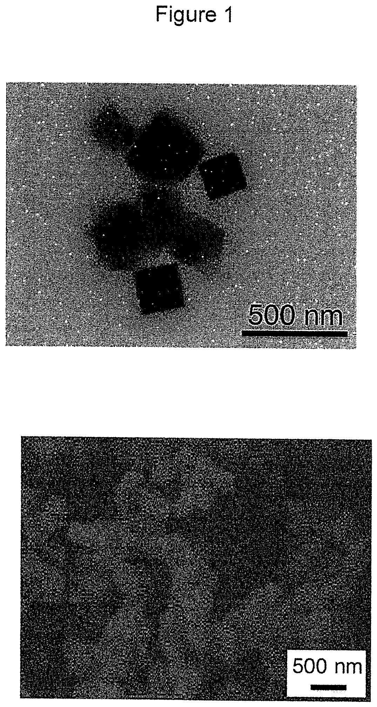

Figure 1 shows SEM and TEM photographs of **UiO-NH₂-P1**. The **UiO-NH₂-P1** was determined to have a roughly 200 nm regular decahedral structure. The size distribution was found to be narrow, and MOF crystals of uniform size and shape were found to have formed.

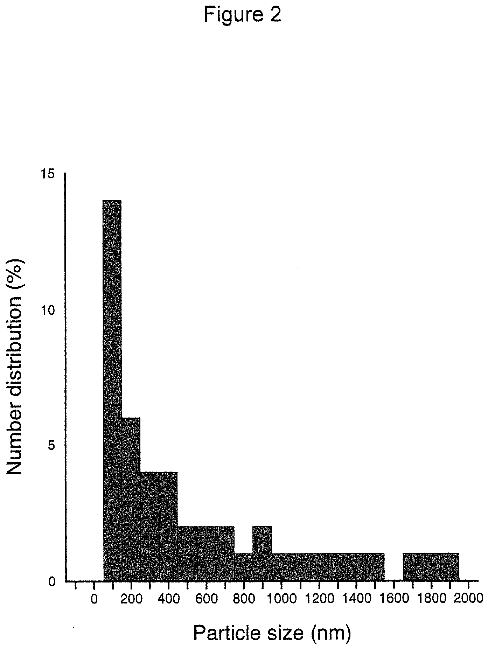

Figure 2 shows the results of DLS measurement (measurement temperature: 25°C, measurement concentration: 5 mg/mL) of **UiO-NH₂-P1** at room temperature. In the same manner as when observed by SEM and TEM, the **UiO-NH₂-P1** was found to have a size of about 150 to 400 nm.

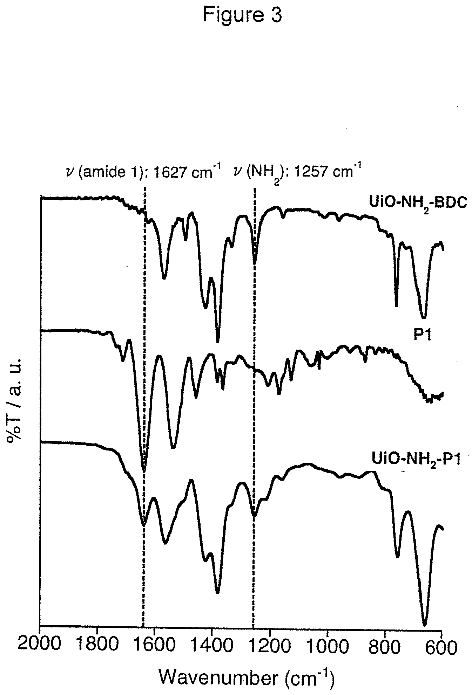

Figure 3 shows an ATR-IR spectrum of **UiO-NH₂-BDC**, an ATR-IR spectrum of **P1,** and an ATR-IR spectrum of **UiO-NH₂-P1**. As shown in Figure 2, a peak derived from P1 (amide 1) was found at 1627 cm⁻¹ from **UiO-NH₂-P1**, and the peak at 1257 cm⁻¹ derived from an amino group was found to remain.

Based on the results of these ATR-IR spectra, an unreacted amino group was found to remain in **UiO-NH₂-P1**.

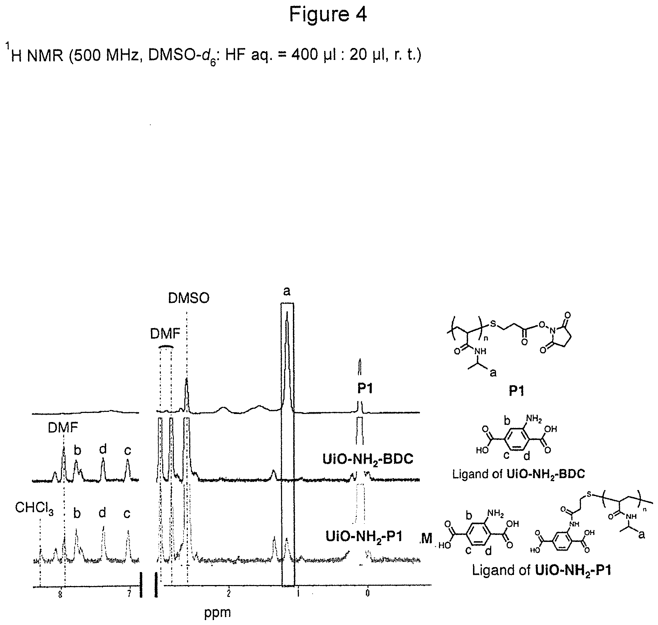

Figure 4 shows a ¹H NMR spectrum of **P1,** a ¹H NMR spectrum of **UiO-NH₂-BDC**, and a ¹H NMR spectrum of **UiO-NH₂-P1** in a mixed solution of DMSO-*d*₆: HF aq. = 400 µL: 20 µL. Based on the ¹H NMR spectrum of **UiO-NH₂-P1**, the presence of a peak derived from the ligands following P1 modification and a peak derived from unmodified ligands were found. Based on the integral ratio of the peak derived after P1 modification, the modification ratio of P1 moieties was about 11 %. This value was one permitting adequate coverage of the **UiO-NH₂**-**BDC** surface with P1. When the MOF is assumed to be a regular octahedron, the length of one side of the MOF to be 200 nm, and the thickness of the lattice structure of the outermost surface to be 14 A, the P1 modification rate (outermost surface coverage modification rate) required to cover the entire outer surface is estimated to be 5.14%.

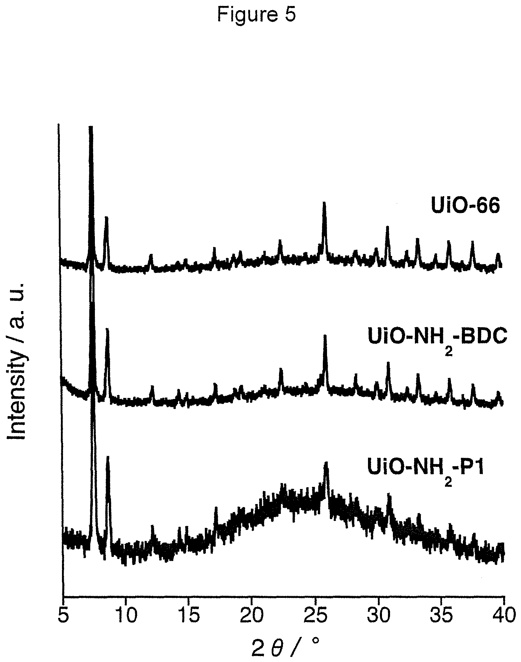

Figure 5 shows an XRPD pattern of UiO-66, an XRPD pattern of **UiO-NH₂-BDC**, and an XRPD pattern of **UiO-NH₂-P1**. Each of these XRPD patterns matched the UiO-66-type diffraction pattern, so the modification reaction was thought to have advanced while maintaining a crystal structure.

### (2) Control of the release of guest molecules enclosed by UiO-NH₂-P1

A 1 mL aqueous solution of 50 mM of a guest molecule (resorufin, caffeine, procainamide) was prepared in a screw tube, **UiO-NH₂-P1** (about 10 mg) was added, and the mixture was left standing for 24 hours. Subsequently, the sample was recovered by centrifugal separation (10,000 rpm, 5 min.) and washed (centrifugal separation: 10,000 rpm, 5 min. 20 times, solvent: water) to prepare a measurement sample. Once the seven-day measurement period had passed, the MOF was decomposed using HF aq. and the absorbance of the guest molecule obtained was normalized as the total amount of enclosed guest molecule.

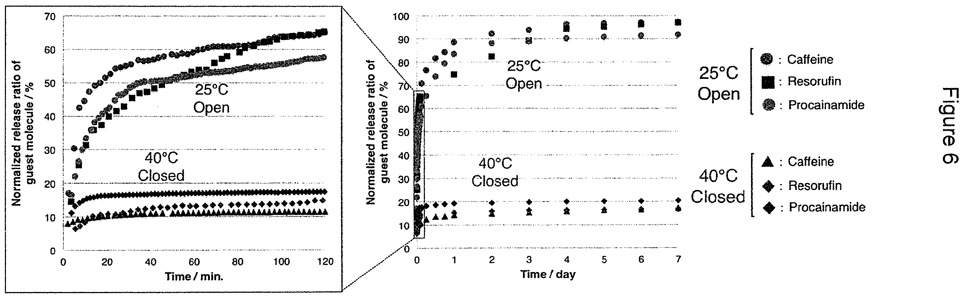

Figure 6 shows the release behavior of resorufin at 25°C and 40°C, the release behavior of caffeine at 25°C and 40°C, and the release behavior of procainamide at 25°C and 40°C based on absorption spectra. In all of these guests, as the measurement period was extended in the 25°C (open phase), the quantity of guest molecules that were released increased. However, no increase in absorbance was seen in the 40°C (closed phase) even when the measurement period was extended.

During a seven-day measurement period, as well, the ability to inhibit release of each of the guest molecules at 40°C (closed phase) was suggested. Thus, the P1 moieties were found to function as gates in response to temperature.

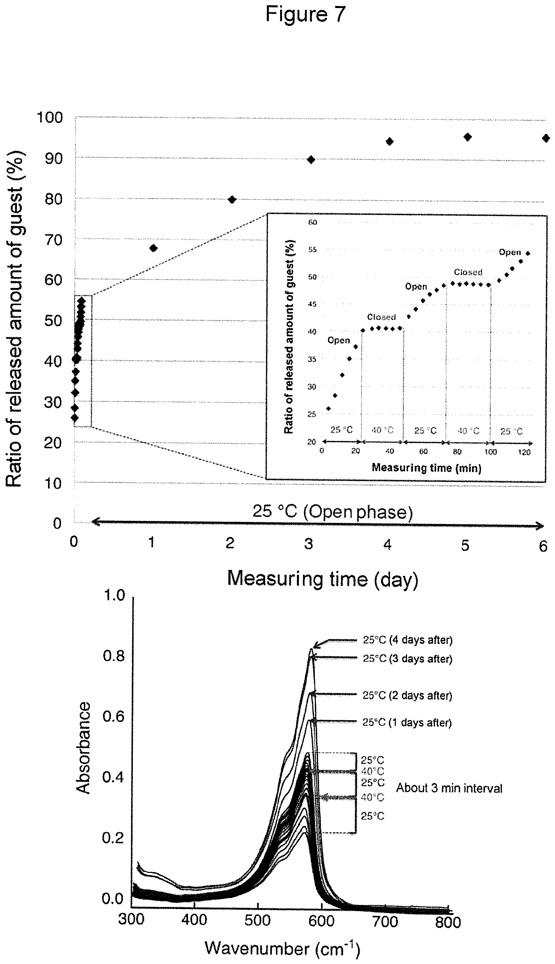

Figure 7 shows the release behavior of a guest molecule in the process of alternating the temperature between 20°C and 40°C at about 20 minute intervals using resorufin as a guest molecule. The release of guest molecules at 25°C (open phase) was confirmed, and the release of guest molecules at 40°C (closed phase) was inhibited, indicating that the release of the guest molecule could be controlled in stages.

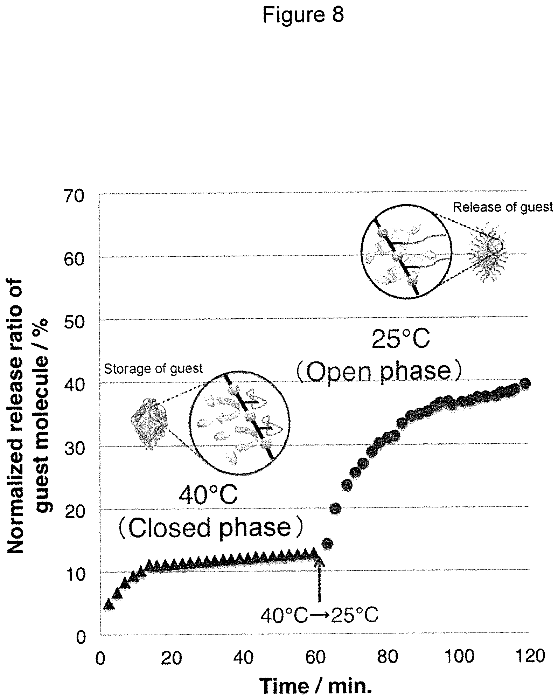

Figure 8 tracks the release behavior of a guest molecule at 40°C in the measurement commencement stage and shows the release behavior of a guest molecule when the temperature was changed to 25°C at 60 minutes from the commencement of measurement using caffeine as guest molecule. While the release of the guest molecule was inhibited at the 40°C stage, release of the guest molecule was found to begin with a change to 25°C.

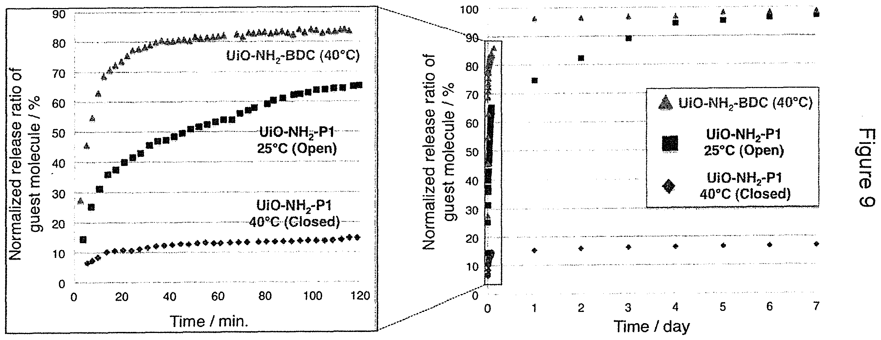

Figure 9 shows the guest molecule release behavior of **UiO-NH₂-BDC** at 40°C, the guest molecule release behavior of **UiO-NH₂-P1** at 25°C, and the guest molecule release behavior of **UiO-NH₂-P1** at 40°C using resorufin as guest molecule. When the guest molecule release behavior of **UiO-NH₂-BDC** at 40°C was compared to the guest molecule release behavior of **UiO-NH₂-P1** at 25°C, modification with P1 was found to slow down the release rate of the guest molecule. Thus, P1 used to modify the surface of **UiO-NH₂-P1** was thought to hinder the release of the guest molecule.

### Example 2

### (1) Synthesis of MOF (UiO-NH2-P2) having P2 moieties

**UiO-NH₂-BDC** (60 mg, about 0.2 mmol: -NH₂) was added to a screw tube, 500 µL of a 0.1 M P2 (820 mg, 0.2 mmol) chloroform solution was added, and the mixture was left standing for 48 hours at 60°C. Subsequently, the MOF was recovered by centrifugal separation (2,000 rpm, 3 min) and washing was conducted with chloroform and methanol. This yielded about 50 mg of the targeted UiO-NH2-P2. The P2 did not dissolve due to protonation in a pH range of less than or equal to pH 4.01, but dissolved due to deprotonation in a pH range of greater than or equal to pH 6.86. The reaction schema is given in Figure 24B.

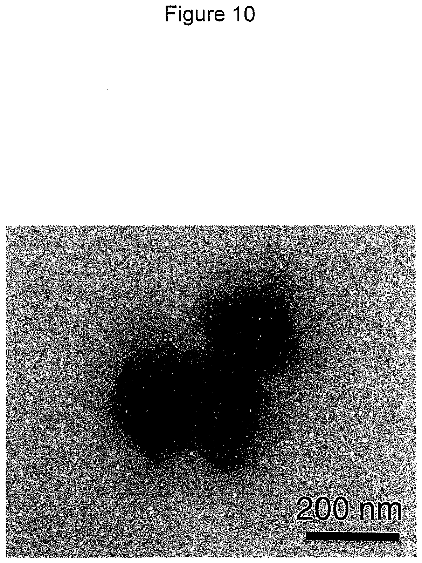

Figure 10 shows a TEM photograph of **UiO-NH₂-P2**. **UiO-NH₂-P2** was found to have formed a regular octahedral structure of about 200 nm on a side. The formation of MOF crystals of narrow size distribution and uniform size and shape was found.

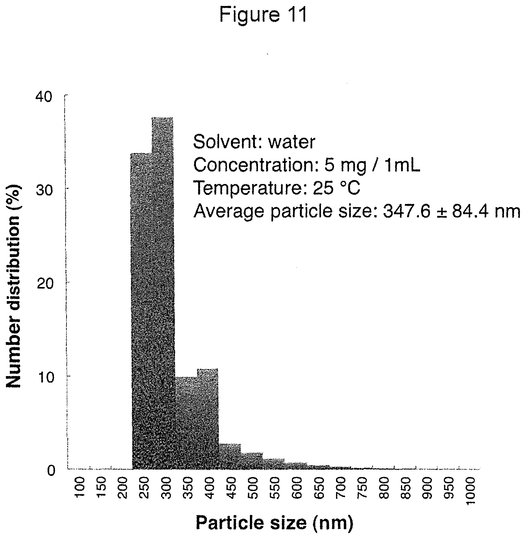

Figure 11 shows the results of DLS measurement (measurement temperature: 25°C, measurement concentration: 5 mg/mL) at room temperature for **UiO-NH₂-P2**. In the same manner as in the results of TEM observation, **UiO-NH₂-P2** was found to have a size of about 200 to 400 nm.

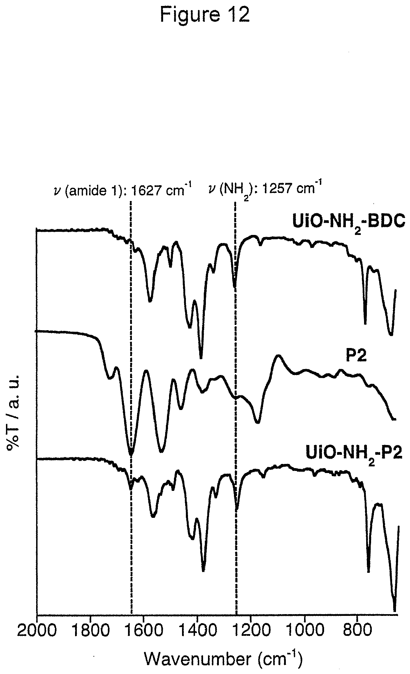

Figure 12 shows an ATR-IR spectrum of **UiO-NH₂-BDC**, an ATR-IR spectrum of **P2,** and an ATR-IR spectrum of **UiO-NH₂-P2**. As shown in Figure 12, a peak derived from P2 (amide 1) was found at 1627 cm⁻¹ from **UiO-NH₂-P2**, with the peak at 1257 cm⁻¹ derived from the amino group remaining.

Based on these ATR-IR spectral results, unreacted amino groups were found to remain in **UiO-NH₂-P2**.

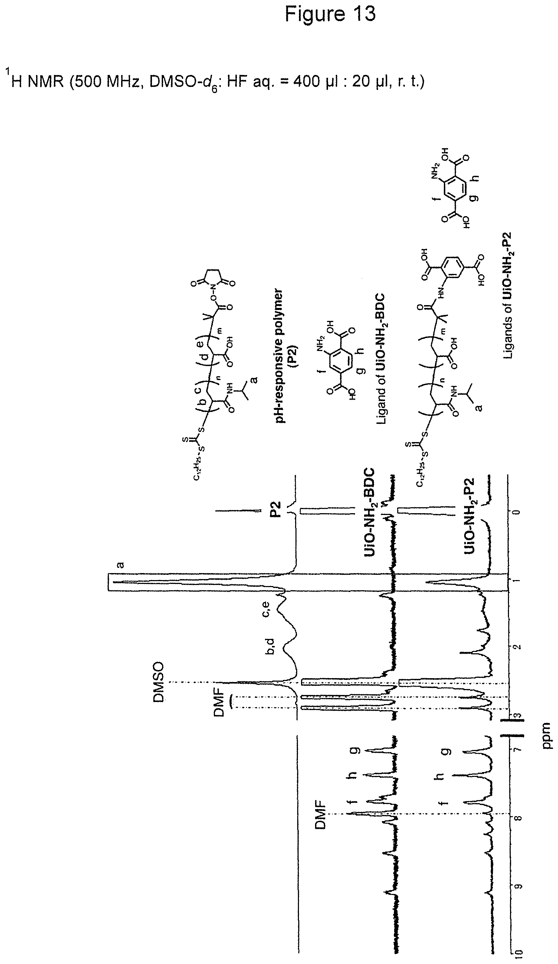

Figure 13 shows a ¹H NMR spectrum of **P2,** a ¹H NMR spectrum of **UiO-NH₂-BDC**, and a ¹H NMR spectrum of **UiO-NH₂-P2** in a mixed solution of DMSO-*d*₆: HF aq. = 400 µL: 20 µL. Based on the ¹H NMR spectrum of **UiO-NH₂-P2**, the presence of a peak derived from the ligands following P2 modification and a peak derived from the unmodified ligands was found. Based on the integral ratio of the derived peak after P2 modification, the modification rate of the P2 moiety was about 5.3%, indicating a value permitting ample coverage of the **UiO-NH₂-BDC** surface. Assuming MOF to be a regular octahedron, the length of one side of MOF to be 346 nm, and the thickness of the lattice structure of the outermost surface to be 14 A, the P2 modification rate (outermost surface coverage modification rate) required to cover the entire outermost surface is estimated to be 3.86%.

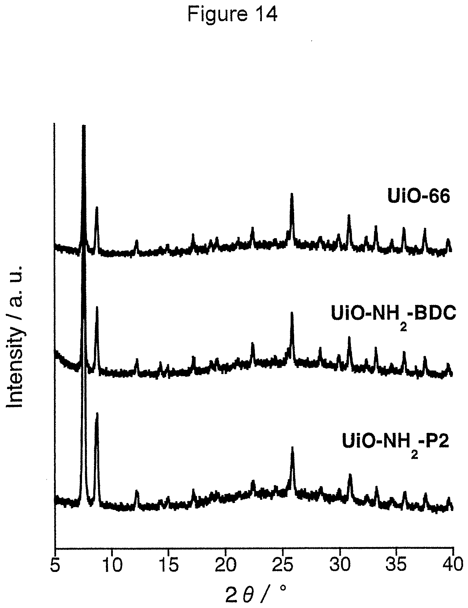

Figure 14 shows an XRPD pattern of **UiO-66**, an XRPD pattern of **UiO-NH₂-BDC**, and an XRPD pattern of **UiO-NH₂-P2**. Since each of the XRPD patterns matched the diffraction pattern of **UiO-66**, the modification reaction was thought to have proceeded while maintaining the crystal structure unaltered.

### (2) Controlling the release of the guest molecule enclosed by UiO-NH₂-P2

A 1 mL aqueous solution (measured for distilled water at pH 7.12) of 50 mM of a guest molecule (procainamide) was prepared in a screw tube, **UiO-NH₂-P2** (about 10 mg) was added, and the mixture was left standing for 24 hours. Subsequently, the sample was recovered by centrifugal separation (10,000 rpm, 5 min.) and washed (centrifugal separation: 10,00 rpm, 5 min. 20 times, solvent: water) to prepare a measurement sample. Once the seven-day measurement period had passed, the MOF was decomposed using HF aq. and the absorbance of the guest molecule obtained was normalized as the total amount of enclosed guest molecule.

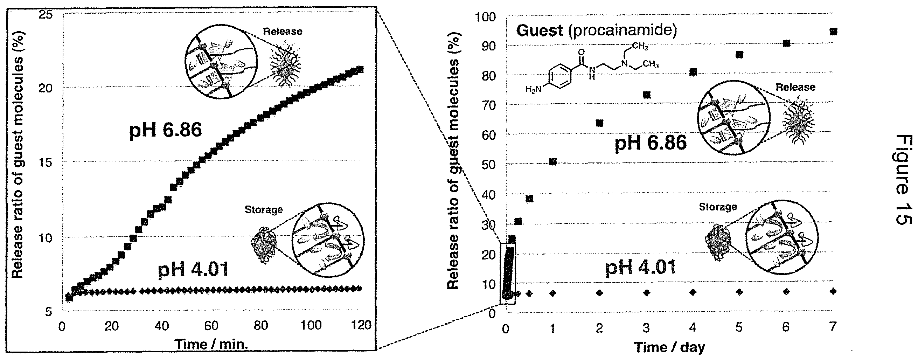

Figure 15 shows the release behavior of procainamide in pH 6.86 and pH 4.01 buffer solutions based on absorption spectra. In the pH 6.86 buffer solution (open phase), the amount of guest molecule that was released increased as the measurement period was extended. In the pH 4.01 buffer solution (closed phase), almost no increase in absorbance was seen despite lengthening of the measurement period.

The fact that it might be possible to control the release of the guest molecule in the pH 4.01 buffer solution (closed phase) was suggested during the seven-day measurement period. Thus, the P2 moiety was found to function as a pH-responsive gate.

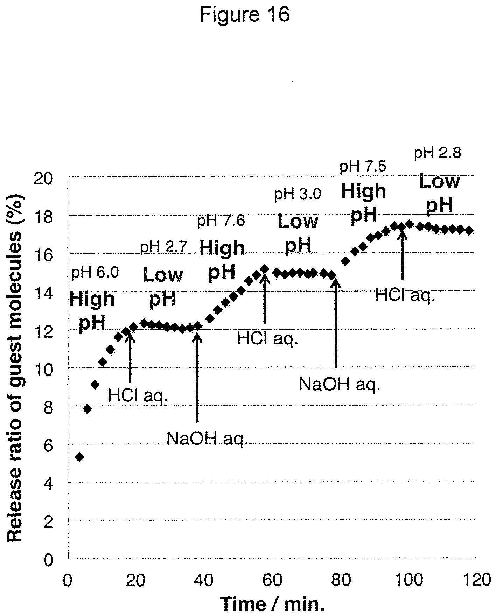

Figure 16 shows the guest molecule release behavior when the pH was alternated between greater than or equal to 7.3 and less than or equal to 4.4 at intervals of about 20 minutes using procainamide as guest molecule. When the pH was greater than or equal to 7.3 (open phase), the guest molecules were found to be released. When less than or equal to pH 4.4 (closed phase), release of the guest molecules was inhibited, indicating that release of the guest molecules could be controlled in stages.

### Example 3

### (1) Synthesis of MOF (UiO-NH₂-P3) having P3 moieties

**UiO-NH₂-BDC** (60 mg, about 0.2 mmol: -NH₂) was added to a screw tube. A 500 µL quantity of a 0.1 M P3 (920 mg, 0.2 mmol) chloroform solution was added, and the mixture was left standing for 48 hours at 60°C. Subsequently, the MOF was recovered by centrifugal separation (2,000 rpm, 3 min.) and washing was conducted with chloroform and methanol. This yielded about 50 mg of the targeted **UiO-NH₂-P3**. Chloroform is a solvent that can always dissolve **P3,** and there is no need to take into account the photoisomerization of **P3** in a chloroform solution. When **P3** was irradiated for 10 seconds with a 4W UV lamp, the azopyridine moiety in **P3** isomerized, the polarity within the molecular increased, and it became soluble in a polar solvent. The reaction schema is given in Figure 24C.

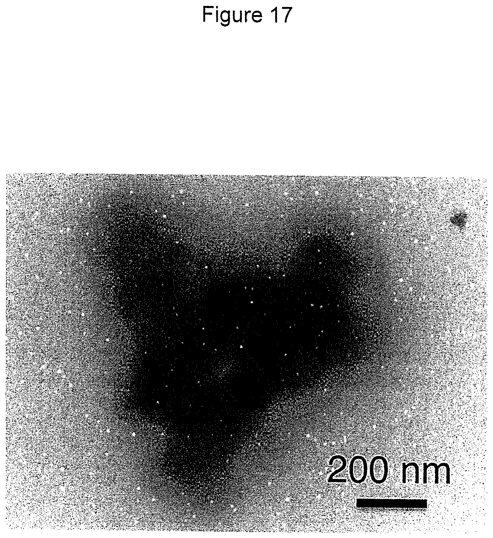

Figure 17 shows a TEM photograph of **UiO-NH₂-P3**. **UiO-NH₂-P3** was found to have formed a regular octahedral structure measuring about 200 nm on a side. The formation of MOF crystals with a narrow size distribution and uniform size and shape was also found.

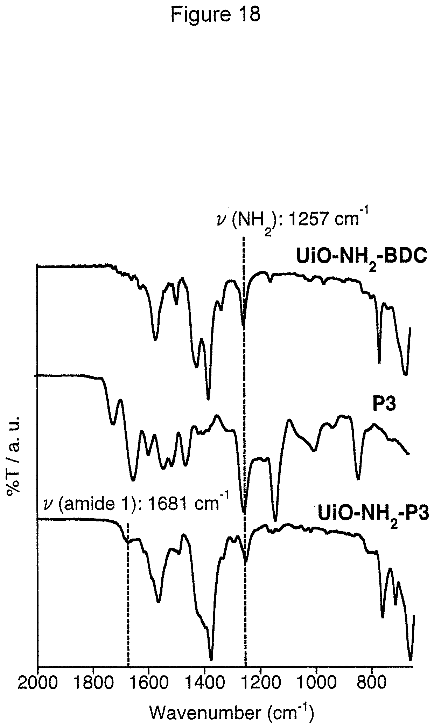

Figure 18 shows an ATR-IR spectrum of **UiO-NH₂-BDC**, an ATR-IR spectrum of **P3,** and an ATR-IR spectrum of **UiO-NH₂-P3**. As shown in Figure 12, a peak derived from **P3** (amide 1) was found at 1681 cm⁻¹ from **UiO-NH₂-P3**, while a peak at 1257 cm⁻¹ derived from an amino group was found to have remained.

Based on these ATR-IR spectral results, unreacted amino groups were found to remain in **UiO-NH₂-P3**.

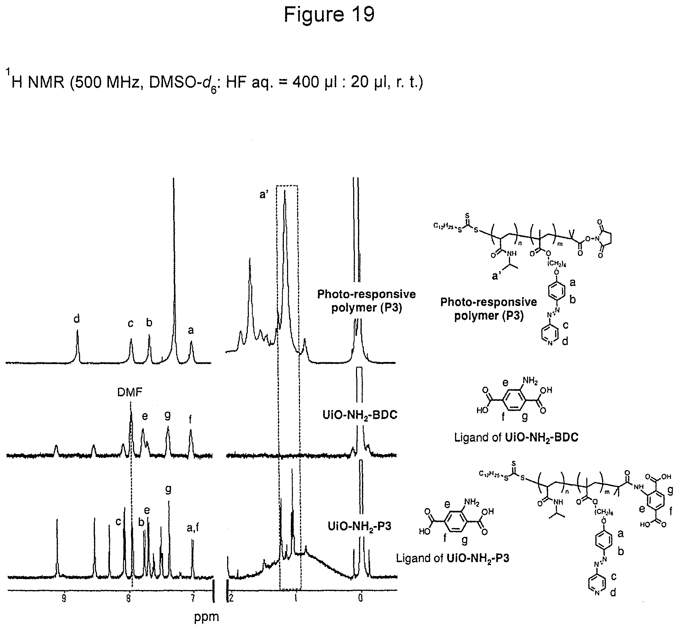

Figure 19 shows a ¹H NMR spectrum of **P3,** a ¹H NMR spectrum of **UiO-NH₂-BDC**, and a ¹H NMR spectrum of **UiO-NH₂-P3** in a mixed solution of DMSO-*d*₆: HF aq. = 400 µL: 20 µL. Based on the ¹H NMR spectrum of **UiO-NH₂-P3**, the presence of a peak derived from the ligands after P3 modification and a peak derived from the unmodified ligands were found. Based on the integral ratio of the derived peak after P3 modification, the P3 moiety modification rate was about 13.2%, a value permitting ample coverage of the surface of **UiO-NH₂-BDC** by **P2.** Assuming the MOF to be a regular octahedron, the length of one side of the MOF to be 200 nm, and the thickness of the lattice structure of the outermost surface to be 14 A, the P1 modification rate (outermost surface coverage modification rate) needed to cover the entire outermost surface was estimated to be 5.14%.

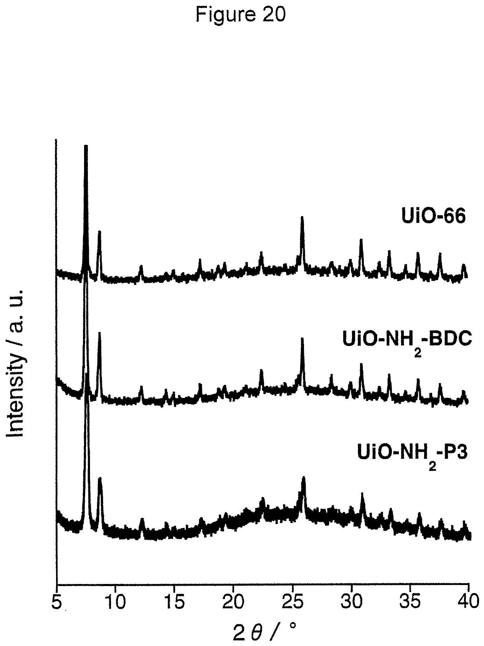

Figure 20 shows an XRPD pattern of UiO-66, an XRPD pattern of **UiO-NH₂-BDC**, and an XRPD pattern of **UiO-NH₂-P3**. All of these XRPD patterns matched the diffraction pattern of UiO-66. Thus, the modification reaction was thought to have proceeded while maintaining the crystalline structure.

### (2) Controlling the release of the guest molecule enclosed by UiO-NH₂-P3

One mL of a 50 Mm guest molecule (resorufin) aqueous solution was prepared in a screw tube, **UiO-NH₂-P3** (about 10 mg) was added, and the mixture was left standing for 24 hours. Subsequently, the sample was recovered by centrifugal separation (10,000 rpm, 5 min) and washed (centrifugal separation: 10,000 rpm, 5 min, 20 times, solvent: water) to prepare a measurement sample. After the seven-day measurement period, the MOF was decomposed in HF aq. and the absorbance of the guest molecule obtained was normalized as the amount of guest molecule enclosed.

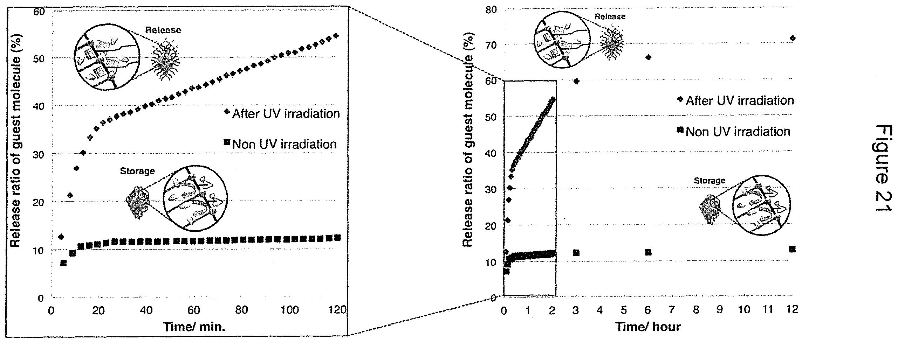

Figure 21 shows the release behavior of resorufin under visible light irradiation and UV irradiation based on absorption spectra. Under UV irradiation (open phase), the quantity of guest molecule released increased as the measurement period was extended. Under visible light irradiation (closed phase), almost no increase in absorbance was observed despite lengthening of the measurement period.

The possibility of inhibiting the release of the guest molecule under visible light irradiation (closed phase) in a 12-hour measurement period was suggested. Thus, the P3 moiety was found to function as a gate that responded to light.

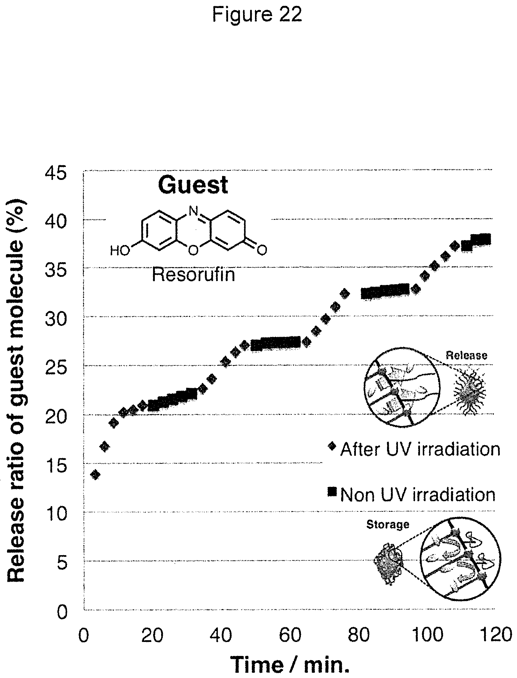

Figure 22 shows guest molecule release behavior with alternating irradiation with visible light and UV radiation at about 20 minute intervals using resorufin as the guest molecule. During UV irradiation (open phase), releasing of the guest molecule was seen. During visible light irradiation (closed phase), releasing of the guest molecule was inhibited. It was thus found possible to control releasing of the guest molecule in stages.

### [Industrial Applicability]

The present invention is useful in fields relating to functional materials having guest molecule trapping and releasing functions.

## Claims

1. A metal-organic framework/stimulus-responsive polymer composite, comprising a metal-organic framework containing organic ligands having at least two functional groups capable of coordinating metal atoms (coordinating functional groups) and metal ions bonding with the coordinating functional groups of the organic ligands, and having a structure in which multiple organic ligands are connected such that one metal ion bonds with two or more coordinating functional groups, and a stimulus-responsive polymer being immobilized on at least a part of a surface of the metal-organic framework.

2. The composite according to Claim 1, wherein the stimulus-responsible polymer is immobilized on the metal-organic framework by bonding with the organic ligands.

3. The composite according to Claim 1 or 2, wherein the stimulus-responsive polymer is a temperature-responsive polymer, pH-responsive polymer, or light-responsive polymer.

4. The composite according to Claim 3, wherein the temperature-responsive polymer is of the lower critical solution temperature (LCST) type having an LCST or of the upper critical solution temperature (UCST) type having an UCST, the pH-responsive polymer is of a type in which a phase transition is induced by a change in pH, and the light-responsive polymer is of a type in which a phase transition is induced in response to light stimulation.

5. The composite according to Claim 4, wherein the LCST-type temperature-responsive polymer is a poly(N-alkylacrylamide), poly(N-alkylmethacrylamide), poly(N-vinylalkylacrylamide), poly(N-vinylmethacrylamide), polyvinyl alkyl ether, polyethylene glycol/polypropylene glycol block copolymer, amino group-comprising acrylic acid ester copolymer or methacrylic acid ester copolymer, polyethylene glycol derivative, acrylic acid ester polymer or methacrylic acid ester polymer comprising an oligoethylene glycol in a side chain, or a polyamino acid; and wherein the UCST-type temperature-responsive polymer is a polyethylene oxide, poly(vinylmethylether), poly(vinylalcohol), poly(hydroxyethyl methacrylate), poly(uracil acrylate), poly(methylacrylamide)/poly(N-acetylacrylamide) copolymer, poly(N-acryloylasparagine amide), poly(N-acryloylglutaminamide), poly(N-acryloylglucinamide), poly(N-methylacryloylasparagine amide), or poly(riboadenylate).

6. The composite according to Claim 3, wherein the pH-responsive polymer is a poly(N-vinylalkylacrylamide), poly(N-alkylacrylamide)/polymethacrylic acid copolymer, poly(N-alkylacrylamide)/polyacrylic acid copolymer, poly(2-ethoxyethylvinylether), polyisobutylvinylether, poly[6-(vinyloxy)hexanoic acid], poly[6-(2-vinyloxyethoxy)]hexanoic acid, or poly[4-(2-vinyloxyethoxy)benzoic acid.

7. The composite according to Claim 3, wherein the light-responsive polymer is a poly(N-vinylalkylacrylamide), poly(N-alkylacrylamide)/poly{6-[4-(4-pyridylazo)phenoxy]hexamethacrylate copolymer, poly(N-alkylacrylamide)/poly[n-(4-phenylazophenyl)acrylamide] copolymer, or poly{4-[2-(vinyloxy)ethoxy]azobenzene}/poly[2-(2-ethoxy)ethoxyethylvinylether] block copolymer.

8. The composite according to any one of Claims 5 to 7, wherein the poly(N-vinylalkylacrylamide) is poly-n-isopropylacrylamide (PNIPAM).

9. The composite of any one of Claims 1 to 8, wherein the modification rate of the surface of the metal-organic framework by the stimulus-responsive polymer falls within a range of greater than 0% and less than or equal to 25%, wherein the modification rate is a molar ratio of the numbers of reactive functional groups present in the organic ligand constituting the metal-organic framework and the stimulus-responsive polymer.

10. The composite according to any one of Claims 1 to 9, wherein the particle diameter of the metal-organic framework falls within a range of from 1 nm to 5 mm.

11. A method for manufacturing a metal-organic framework/stimulus-responsive polymer composite, comprising the steps of:
preparing a metal-organic framework comprising organic ligands that have at least two functional groups capable of coordinating metal atoms (coordinating functional groups) and reactive functional groups being reactive with the reactive functional groups present in a temperature-responsive polymer, and metal ions that bond with the coordinating functional groups of the organic ligands, and having a structure in which multiple organic ligands are connected such that one metal ion bonds with two or more coordinating functional groups; and
subjecting the stimulus-responsive polymer comprising reactive functional groups and the metal-organic framework to conditions permitting reaction of the reactive functional groups present in the stimulus-responsive polymer with the reactive functional groups present in the organic ligands in the metal-organic framework to obtain a metal-organic framework/stimulus-responsive polymer in which the stimulus-responsive polymer is immobilized on at least a part of a surface of the metal-organic structure.

12. The manufacturing method according to Claim 11, in the form of a method for manufacturing the composite according to any one of Claims 1 to 10.

13. A method for manufacturing a guest molecule-containing metal-organic framework/temperature-responsive polymer composite including the capturing and sealing in of guest molecules into the metal-organic framework/temperature-responsive polymer composite according to any one of Claims 1 to 10 (wherein the temperature-responsive polymer is of the lower critical solution temperature (LCST) type having a LCST), comprising the steps of:
obtaining a metal-organic framework/temperature-responsive polymer composite in which guest molecules have been captured by immersing the metal-organic framework/temperature-responsive polymer composite in a solvent in which guest molecules have been dissolved or dispersed, at a temperature that is lower than the LCST of the temperature-responsive polymer; and
exposing the metal-organic framework/temperature-responsive polymer composite in which the guest molecules have been captured to a temperature exceeding the LCST of the temperature-responsive polymer to seal the incorporated guest molecules within the metal-organic framework/temperature-responsive polymer composite and obtain a guest molecule-containing metal-organic framework/temperature-responsive polymer composite.

14. A method for manufacturing a guest molecule-containing metal-organic framework/temperature-responsive polymer composite including the capturing and sealing in of guest molecules into the metal-organic framework/temperature-responsive polymer composite according to any one of Claims 1 to 10 (wherein the temperature-responsive polymer is of the upper critical solution temperature (UCST) type having an UCST), comprising the steps of:
obtaining a metal-organic framework/temperature-responsive polymer composite in which guest molecules have been captured by immersing the metal-organic framework/temperature-responsive polymer composite in a solvent in which guest molecules have been dissolved or dispersed, at a temperature that is higher than the UCST of the temperature-responsive polymer; and
exposing the metal-organic framework/temperature-responsive polymer composite in which the guest molecules have been captured to a temperature less than or equal to the UCST of the temperature-responsive polymer to seal the incorporated guest molecules within the metal-organic framework/temperature-responsive polymer composite and obtain a guest molecule-containing metal-organic framework/temperature-responsive polymer composite.

15. A method for manufacturing a guest molecule-containing metal-organic framework/pH-responsive polymer composite including the capturing and sealing in of guest molecules into the metal-organic framework/pH-responsive polymer composite according to any one of Claims 1 to 10, comprising the steps of:
obtaining a metal-organic framework/pH-responsive polymer composite in which guest molecules have been captured by immersing the metal-organic framework/pH-responsive polymer composite in a solvent in which guest molecules have been dissolved or dispersed in a pH range that dissolves the pH-responsive polymer; and
exposing the metal-organic framework/pH-responsive polymer composite in which the guest molecules have been captured to a pH range at which the pH-responsive polymer is insoluble to seal the incorporated guest molecules within the metal-organic framework/pH-responsive polymer composite and obtain a guest molecule-containing metal-organic framework/pH-responsive polymer composite.

16. A method for manufacturing a guest molecule-containing metal-organic framework/light-responsive polymer composite including the capturing and sealing in of guest molecules into the metal-organic framework/light-responsive polymer composite according to any one of Claims 1 to 10, comprising the steps of:
obtaining a metal-organic framework/light-responsive polymer composite in which guest molecules have been captured by immersing the metal-organic framework/light-responsive polymer composite in a solvent in which guest molecules have been dissolved or dispersed under ultraviolet radiation irradiation that dissolves the light-responsive polymer; and
exposing the metal-organic framework/light-responsive polymer composite in which the guest molecules have been captured to visible light irradiation conditions under which the light-responsive polymer is insoluble to seal the incorporated guest molecules within the metal-organic framework/light-responsive polymer composite and obtain a guest molecule-containing metal-organic framework/light-responsive polymer composite.

17. The method according to any one of Claims 13 to 16, wherein the guest molecules are the active ingredient of a treatment drug, preventive drug, or test agent.
